(19) Europäisches Patentamt
European Patent Office
Office européen des brevets

(11) EP 2 865 394 A1

# (12) EUROPEAN PATENT APPLICATION

published in accordance with Art. 153(4) EPC

(43) Date of publication:
**29.04.2015 Bulletin 2015/18**

(21) Application number: **13806520.6**

(22) Date of filing: **21.06.2013**

(51) Int Cl.:
***A61K 49/14*** (2006.01) ***A61K 49/18*** (2006.01)
***G01N 33/53*** (2006.01)

(86) International application number:
**PCT/ES2013/070407**

(87) International publication number:
**WO 2013/190165 (27.12.2013 Gazette 2013/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.06.2012 ES 201230972**

(71) Applicant: **Consejo Superior De Investigaciones Científicas (CSIC)**
**28006 Madrid (ES)**

(72) Inventors:
- **VELEZ TIRADO, Marisela**
  **28049 Madrid (ES)**
- **DAICH, Julián**
  **28049 Madrid (ES)**

(74) Representative: **Illescas, Manuel**
**Manuel Illescas y Asociados, S.L. (MIA)**
**Principe de Vergara n° 197**
**Oficina 1°A**
**28002 Madrid (ES)**

## (54) COMPOUNDS HAVING MAGNETIC FUNCTIONALITY, IMPLANTS OR GELS DERIVED FROM SAME, AND USE OF BOTH IN ORDER TO DETERMINE THE ENZYME ACTIVITY OF AN ENZYME

(57) The invention relates to an implant or gel including at least one compound having magnetic functionality, which in turn includes a substrate of at least one enzyme involved in regenerating the extracellular matrix and a plurality of magnetic particles. The invention also relates to a compound having magnetic functionality which includes a substrate of at least one enzyme involved in regenerating the extracellular matrix and a plurality of magnetic particles, preferably with no coating or surface modification, or functionalised with carboxyl groups, for the production of an implant or gel for monitoring an enzymatic activity involved in regenerating the extracellular matrix. The substrate is preferably selected from the group that comprises collagen, chondroitin sulphate and hyaluronic acid, and the magnetic particles are superparamagnetic particles.

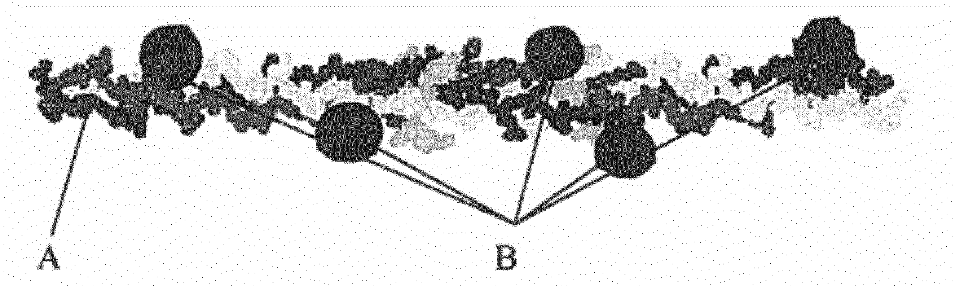


Fig. 4

EP 2 865 394 A1

## Description

### FIELD OF THE INVENTION

This invention relates in general to an implant or gel comprising at least a compound with magnetic functionality. These implants or gels can be used for measuring the enzymatic activity associated with regeneration of the extracellular matrix by measuring the variation in the magnetic properties of at least one of the compounds with magnetic functionality, in the magnetic properties of one or more of its degradation products, or in both.

In addition, this invention also relates to new compounds with magnetic functionality comprising a substrate of at least one enzyme, preferably collagen, chondroitin sulphate or hyaluronic acid, and a plurality of magnetic particles without coating or modification of surface, or functionalised with carboxylic groups.

### BACKGROUND OF THE INVENTION

Enzymes are proteins catalysing chemical reactions. In enzyme reactions, the molecules on which an enzyme acts are called substrates and are transformed by the enzymes into different molecules called metabolites. Enzymes are generally very specific for the reactions and substrates that they catalyse.

The importance of enzymes is shown by the fact that a fatal disease can be caused by the malfunction of a single type of enzyme of the thousand types present in the body.

Several clinical conditions, such as unstable plaque, metastasis or variations in tissue regeneration are related to enzyme disorders.

Most biological material is diamagnetic; in other words, it has a negative magnetic susceptibility. The magnetic particles bound to a macromolecule of biological origin can cause a magnetic signature different from that of the macromolecule of biological origin. This magnetic signature is based on the composition, size and the shape of the particles. Magnetisation of a given material is defined as its mean magnetic moment per unit of volume and is determined by the internal structure and composition of this material. Examples of magnetic particles include paramagnetic, superparamagnetic and ferromagnetic particles, which are commonly used as contrast agents in the form of individual particles, microencapsulated in matrices or bound to other molecules. These magnetic contrast agents are designed to keep their magnetic properties constant throughout the measurements and cause image effects according to their concentration and distribution in the different tissues and compartments of the body. Some factors in the distribution of magnetic particles, such as aggregation or coatings in the surface, can affect the contrast effects that they cause.

Currently the detection and/or follow-up of enzyme processes that can be related to disorders or diseases are usually performed by biopsies, extractions, or indirect methods such as estimation of enzyme and metabolite levels. There is today a need for developing a product which allows the follow-up of the enzymatic activity *in situ*, without needing to take a sample from the body under study, using non-invasive detection methods that leave the body untouched.

In this patent application a "compound with magnetic functionality" is defined as that with the ability to suffer conformational or structural changes leading to a measurable change in its magnetic properties, in the magnetic properties of one or more of its degradation products, or in both.

The patent application US 2011/0182815 A1 (Daich, J.) describes diagnostic methods using substrates modified to have magnetic functionality. These substrates are obtained from the combination thereof with magnetic particles such that since said substrates have magnetic functionality and are digested by the enzymes of interest a measurable change occurs in the distribution of magnetic particles. However, patent application US 2011/0182815 A1 only mentions pharmaceutical formulations that include intravenous and encapsulation methods as delivery forms for these substrates modified to have magnetic functionality that are not efficient for reaching some types of connective tissue, such as various joint cartilages. The examples of this patent application only mention the use of some proteins as substrates for obtaining compounds with magnetic functionality, leaving aside the use of other elements such as glycosaminoglycans that are involved in and fulfil functions such as hydration, flexibility and promotion of regeneration of the extracellular matrix. These functions are significantly important for the extracellular matrix and also for the connective tissue. The methods for modifying substrates to obtain compounds of this application are limited to the existence of carboxyl groups in the former, thus excluding a high number of substrates that do not have said carboxyl groups available for modification. The latter include the glycosaminoglycans discussed above.

On the other hand, the compounds with magnetic functionality and the methods for obtaining them described in patent application US 2011/0182815 A1 are based on the use of magnetic particles with surface modifications, which enhance anchorage of said magnetic particles to the substrates of interest. Said surface modifications are performed with diamagnetic materials, leading to a reduction in the intensity and scope of the magnetic field that these particles with surface modifications generate in their setting. Therefore, when adding said surface modifications to the magnetic particles the interaction between the magnetic moments of the different particles can decrease, and these events are disadvantageous for the type of measurements used in detection methods such as the magnetic resonance imaging proposed in the aforementioned US patent application.

Patent application WO 2011/075476 A1 (Bennet K., 2011) describes methods for evaluating the molecular structure of biocompatible hydrogels using magnetic resonance imaging (MRI) techniques. To that end, it describes gels comprising biocompatible hydrogen and a magnetic contrast agent. This gel can be implanted *in vivo* so that the disintegration of magnetic particles comprised in the contrast agent, as a result of the interaction of the hydrogel with the extracellular matrix cells or the enzyme action can be measured by MRI. In particular, this document discloses the use of cationised ferritin as contrast agent. This compound binds to the hydrogel through the anion groups present in the structure of the aforementioned hydrogel. In this application, unlike the present application, the term "aggregation" is used recurrently to define the immobilisation or fixation of particles in the gel, which does not involve the formation of particle aggregates as such.

Furthermore, the addition of an enzyme precursor to the gel for extending the gel breakdown speed is described. However, as this breakdown is the result of a cascade reaction of the activation of a pre-implanted precursor of an enzyme and not of the target enzyme, this method loses precision in intensity and duration of the enzymatic activity object of study.

## BRIEF DESCRIPTION OF THE INVENTION

The present invention relates to an implant or a gel comprising at least a compound with magnetic functionality comprising in turn a substrate of at least one enzyme involved in the regeneration of the extracellular matrix and a plurality of magnetic particles.

In the patent application US 2011/0182815 A1 the use of modified substrates to manufacture an implant or gels is not mentioned or suggested, and the use of said implant in monitoring enzymatic activity in connective tissue is not mentioned or suggested either. Unlike the disclosure contained in this US patent application, the use of implants or gels allows the enzymatic reaction to take place in the same implant or gel, i.e., on a localised predetermined basis.

Several types of connective tissue implants exist today. These implants usually include biocompatible materials, and can be even extractions of tissues from living beings or produced by culture techniques. The therapeutic applications of these implants are highly diverse including, for instance, the substitution in the regeneration of connective tissue in joints or orthodontics. The main therapeutic actions of these implants are to support, replace or cause the regeneration or the recipient tissue. There is today no non-invasive method for in situ follow-up of the integration of internal implants or regeneration of their constituents by components generated by the recipient body.

On the other hand, the gel comprising at least a compound with magnetic functionality as described in this patent application is adequate for analysis in vitro and can include additionally the same biocompatible materials as an implant. Said gels are commonly used to mimic the environment of the extracellular matrix and have varied applications in research and diagnosis.

In another aspect, the present invention relates to a compound with magnetic functionality comprising a substrate of at least one enzyme involved in the regeneration of the extracellular matrix and a plurality of magnetic particles for manufacturing an implant or a gel to monitor an enzymatic activity involved in the regeneration of the extracellular matrix in a human being or another living being.

In addition, this invention also relates to the use of said compound with magnetic functionality to manufacture an implant or a gel to monitor an enzymatic activity involved in the process of regeneration of the extracellular matrix in a human being or a living being. After placing the implant described in this invention, the compound with magnetic functionality comprised in it can react with at least one enzyme involved in the regeneration of the extracellular matrix, giving rise to a measurable variation in its magnetic properties, in the magnetic properties of one or more of its degradation products, or in both. Therefore, the present invention provides an implant which allows monitoring the regeneration of the extracellular matrix in said implant without needing to perform biopsies.

This invention relates also to a compound with magnetic functionality comprising a substrate of at least one enzyme and a plurality of magnetic particles, this compound characterised in that:

i) the magnetic particles do not have any coating or modification of surface,
or
ii) the magnetic particles are functionalised with carboxyl groups.

After the administration of the compound with magnetic functionality of the present invention, this compound can react with at least one enzyme leading to a measurable variation in its magnetic functionality. Therefore, the compound with magnetic functionality of this invention can be used for the detection of an enzymatic activity, in particular that involved in the regeneration of the extracellular matrix.

A further object of this invention is also to provide an advantageous method for obtaining a compound with magnetic functionality wherein the magnetic particles do not have any coating or modification of surface, this method comprising:

a) modifying the substrate of at least one enzyme, and
b) immobilising a plurality of magnetic particles that do not have any coating or modification of surface in the compound modified obtained in stage a).

Unlike other methods known in the state of the art where the magnetic particles are coated or have some modification of surface, the method for obtaining a compound with magnetic functionality of this invention allows binding the substrate of the enzyme to magnetic particles that are almost entirely magnetic nucleus.
In preferred embodiments of this invention, the magnetic particles are superparamagnetic particles.
In other preferred embodiments of the present invention, the substrate can be selected from the group consisting of protein, modified protein, polypeptide, modified polypeptide, glycoprotein, glycosaminoglycan and mixtures thereof, being more preferable that said substrate is selected from the group consisting of collagen, chondroitin sulphate and hyaluronic acid.
In addition, the present invention also relates to a compound with magnetic functionality comprising a substrate of at least one enzyme and a plurality of magnetic particles that have no coating or modification of surface, where said compound with magnetic functionality is obtained by the method described in this patent application. Preferably, the magnetic particles are nanoparticles of magnetite, and the substrate can be selected from the group consisting of collagen, chondroitin sulphate and hyaluronic acid.
The present invention also relates to a product comprising at least a compound with magnetic functionality comprising also a substrate of at least one enzyme and a plurality of magnetic particles, where

i) the magnetic particles do not have any coating or modification of surface, or
ii) the magnetic particles are functionalised with carboxylic groups as described in this patent application, where said product is selected from the group that consists of an implant, a gel, a pharmaceutical composition and a contrast agent.

Preferably, the present invention relates to a product that is selected from the group consisting of an implant and a gel, and this comprises at least a compound with magnetic functionality that comprises in turn a substrate with a least one enzyme involved in the process of cell regeneration and a plurality of magnetic particles, where i) the magnetic particles do not have any coating or modification of surface, or ii) the magnetic particles are functionalised with carboxylic groups as described in this patent application. In an even more preferred embodiment, the substrate is selected from the group consisting of collagen, chondroitin sulphate, and hyaluronic acid, and the magnetic particles are magnetite nanoparticles without coating or modification of surface.
The present invention also relates to the use of the compound with magnetic functionality that in turn comprises a substrate with at least one enzyme and a plurality of magnetic particles, where i) the magnetic particles do not have any coating or modification of surface, or ii) the magnetic particles are functionalised with carboxylic groups as described in this patent application, to manufacture a product as described in this patent application to establish the enzymatic activity of one or more enzymes. Preferably, to detect a disease or a disorder associated with the enzymatic activity of at least one enzyme.
In addition, the present invention also relates to the use of the compound with magnetic functionality comprising in turn a substrate of at least one enzyme and a plurality of magnetic particles, where i) the magnetic particles do not have any coating or modification of surface, or ii) the magnetic particles are functionalised with carboxylic groups as described in this patent application, to manufacture the implant or the gel as described in this patent application to monitor an enzymatic activity involved in the regeneration of the extracellular matrix in a human being or another living being.

## DETAILED DESCRIPTION OF THE INVENTION

This invention uses, amongst other principles, the fact that the properties of magnetic materials are derived from the distribution and shape of their components. If any of these two characteristics can be modified by the enzymatic activity, it can be detected based on the measurement of the magnetic properties. A form to do this is to provoke particle aggregation as a result of the enzymatic activity. To understand the effect of magnetic particle aggregation first a general description is given of the properties of the different types of magnetic materials that can form them.
Ferromagnetic materials in general contain transition metals such as for instance iron (Fe), magnesium (Mg), manganese (Mn), cobalt (Co), nickel (Ni), zinc (Zn), and copper (Cu). Ferromagnetic materials are also characterised in that they are formed by groups of about $10^{17}$ to $10^{21}$ atoms called magnetic domains, all of them having their magnetic moments (or spin populations) pointing in the same direction. The magnetic moments of the domains are oriented at random in non-magnetised materials and point in preferred directions in magnetised materials. The capacity of ferromagnetic materials to continue to be magnetised when an external magnetic field is removed is a unique factor as compared to paramagnetic, superparamagnetic and diamagnetic materials that lose their preferred magnetisation orientation in similar circumstances.

Some paramagnetic materials can show a permanent, stable magnetisation after removing the magnetic field if they are below the so-called Curie temperature, a transition temperature below which spins are aligned in a stable manner with or against the magnetic field. These materials are called antiferromagnetic and show a resulting magnetisation since both orientations of the spins are not cancelled. Above the Curie temperature, as the interaction of spins is weak when removing the external magnetic field, the thermal motion is sufficient for the spins to acquire a random orientation and thus eliminate the permanent magnetisation. All iron particles formed based on magnetite have ferrimagnetic or ferromagnetic properties at physiological temperatures (the transition temperature of iron particles is close to 850 Kelvin).

Superparamagnetic materials can be formed by individual domains of materials that in bulk have ferromagnetic properties. Their magnetic susceptibility, the magnetisation degree of a material in response to a magnetic field, is between that of ferromagnetic and paramagnetic materials. Superparamagnetic materials behave as ferromagnetic materials under the effects of external magnetic fields, but they have paramagnetic characteristics in the absence of an external magnetic field. These superparamagnetic materials are generally formed by nanometric crystalline particles and macroscopically have ferromagnetic properties or properties of nanocomposite magnets.

The concept of anisotropy is important to understand superparamagnetism. The spins of a high number of paramagnetic ions arranged in order interact so that, when placed in an external field, the resulting magnetisation is oriented in all directions, i.e., is isotropic. When spin coupling is aligned in any direction in relation to an external field then it is said that magnetisation is anisotropic. For instance, for magnetite crystals there are six orientation axes. Therefore, under the effect of a magnetic field, the result would be six orientations, each with a different energy value. The minimum energies occur when the orientation is parallel to the external field and maximum when the orientation is antiparallel.

Temperature has a destabilising effect on the magnetism of superparamagnetic materials. Thermal energy avoids alignment of the magnetic moments present in the superparamagnetic particles. After removal of an applied magnetic field, the magnetic moments of the superparamagnetic materials still exist, but the fast motion induced by the temperature misaligns them. At the temperatures of biological systems and in the magnetic fields applied during magnetic resonance imaging (MRI) superparamagnetic materials are less magnetisable than their ferromagnetic homologues.

Some magnetic properties, for instance, ferro or ferri magnetism arise from the cooperative behaviour of domains in macroscopic scales and they are not intrinsic to the molecules or ions forming the material. These properties are the consequence of the interactions between molecules or ions and, therefore, the methods to predict, control and modulate the solid state structure are relevant for understanding and managing this behaviour.

Some nanomaterials, such as magnetite nanoparticles in colloidal suspension, show properties different from those in solid state in macroscopic scales, either in bulk o as individual particles. For the materials formed by large crystals, of diameters greater than 15 nm, the spins are aligned and subdivided in small magnetic domains called Weiss domains. The directions of the different spins tend to align in the same direction along the anisotropic axes; this leads to reaching a minimum value of the anisotropy energy and the system can be considered isotropic. This explains why ferromagnetic crystals, for instance, magnetite, must be magnetised by placing them in an external field for the purpose of obtaining the permanent magnetism. If the crystals of magnetic material are smaller than the Weiss domains, superparamagnetism can be observed. For the spin to pass from one anisotropic axis to another, an energy entry equal to the desired transition is required. The anisotropy energy is known as KV, where K is the anisotropy constant and V is the crystal volume. The distance between several crystalline particles influences the KV value, as crystals which are very close to each other can allow interactions between the spins that cause a KV increase. The turn of directions of the spins along the axis of an applied external field is defined as the Néel relaxation time, and is the result of the thermal agitation of the crystals. Néel relaxation ($T_N$) can be expressed as follows:

$$\tau_N = \tau_0 e^{\frac{KV}{kT}}$$

Where k is the Boltzmann constant, T is the constant temperature of the medium and $T_0$ is a constant. For large crystals, the Néel relaxation time is very long and the magnetic moments can be considered as blocked to the easy anisotropic axis. For crystals smaller than 6 nm in diameter, the transmissions are fast, in the order of nanoseconds. For small ferromagnetic crystals in liquid media, not only the Néel relaxation time modulates the fluctuations, but also the movement of the crystals. The Brownian or mean rotation time of the system is equal to:

$$\tau_r = \frac{4\pi\alpha 3\eta}{3kT}$$

Where $\alpha$ is the radius of the particle and $\eta$ the viscosity of the liquid. For a system of paramagnetic crystals, the resulting magnetisation is given by the following relationship:

$$M = M_{sat} \cdot L(\alpha), \quad L(\alpha) = \coth(\alpha) - \frac{1}{\alpha}, \quad \alpha = \mu\frac{B_0}{kT}$$

Where $B_0$ is the external magnetic field, $\mu$ is the total magnetic momentum of the crystal and $M_{sat}$ defines the field of closure of magnetisation or saturation along the low-energy axis. The superparamagnetic particles have magnetic moments much higher than the individual moments associated with the ions forming the crystals for the fact that, in the absence of an external field, the mean magnetic moment of the particles is zero due to the average of the Néel relaxation of the crystals and the particle rotation.

This invention is based on the fact that the magnetic behaviour of the magnetic nanoparticles depends on the distance between them and that that difference is measureable experimentally. The distance between these magnetic nanoparticles immobilised in a matrix or in a macromolecule depends on the integrity of this support. If this integrity is affected by the effect of a hydrolytic effect, for instance, and if for said effect the magnetic particles will be aggregated this will result in a magnetic signal different from that obtained from the particles present in a matrix or unchanged molecule.

This invention relates to an implant or a gel comprising at least a compound with magnetic functionality comprising also a substrate of at least one enzyme involved in regeneration of the extracellular matrix and a plurality of magnetic particles, with the condition that, when the implant or gel comprises more than one compound with magnetic functionality, these compounds are placed in different regions.

Preferably, when the implant or gel comprises more than one compound with magnetic functionality as described in this patent application, these are arranged in separate areas of the implant or gel. The use of an implant or gel comprising more than one compound with magnetic functionality, each comprising a different substrate, can allow a simultaneous follow-up of different enzymes.

For the purpose of this invention, a "plurality of magnetic particles" is defined as more than one non-diamagnetic particle, defining also "particle" as a combination of molecules or ions, preferably ordered as a crystal, of micrometric or smaller size, preferably with a size under 100 nm in diameter.

On the other hand, in this invention "a compound with magnetic functionality" is defined as the compound with the ability to cause conformational or structural changes leading to a measurable variation in its magnetic properties, the magnetic properties of one or more than its degradation products or in both.

The compounds with magnetic functionality must preserve their functional properties during the preparation of the gel or exposure to physiological conditions. This can be achieved through binding of magnetic particles, more preferably magnetic nanoparticles, with substrates through covalent bonds during the preparation of said compounds with magnetic functionality. In some preferred embodiments of the invention wherein said magnetic particles tend to aggregate, the preparation of compounds with magnetic functionality by covalent bonds is particularly advantageous.

In a preferred embodiment, the implant or gen of the present invention comprises a compound with magnetic functionality that also comprises a plurality of magnetic particles bound by covalent bonds to the substrate of at least one enzyme involved in the regeneration of the extracellular matrix.

In another preferred embodiment, the present invention relates to the implant or gel comprising a compound with magnetic functionality that in turn comprises a substrate of at least one enzyme involved in the regeneration of the extracellular matrix and a plurality of magnetic particles, as described in this patent application.

In another preferred embodiment, the present invention relates to the implant or gel comprising at least a compound with magnetic functionality that in turn comprises a substrate of at least one enzyme involved in the regeneration of the extracellular matrix and a plurality of magnetic particles as described in this patent application, where the magnetic particles are superparamagnetic particles. Preferably, the superparamagnetic particles can be nanoparticles of ferrites, for instance, magnetite, maghemite, cobalt ferrites, silver or magnesium.

These preferred magnetic particles allow the covalent bonds with the substrate. In addition, they can be aggregated at physiological pH.

In an even more preferred embodiment, the superparamagnetic particles are magnetite nanoparticles, and it is particularly preferable that the magnetite nanoparticles have a diameter of less than 15 nm.

In another preferred embodiment, the present invention relates to the implant or gel that comprises at least a compound with magnetic functionality that in turn comprises a substrate of at least one enzyme involved in the regeneration of the extracellular matrix and a plurality of magnetic particles, as described in this patent application, where the substrate can be selected from the group consisting of protein, modified protein, polypeptide, modified polypeptide, glycoprotein, glycosaminoglycan and a combination thereof.

Preferably, this substrate reacts with at least one enzyme that is a metal dependent enzyme. In a more preferred embodiment, this enzyme is selected from a group consisting of a protease, an oxidase and a sulphatase. Even more preferably, this enzyme is selected from the group consisting of hyaluronidase and a metalloprotease.

In a particularly preferred embodiment, the substrate comprised in the compound with magnetic functionality described in this patent application reacts with at least one enzyme that is a matrix metalloprotease (MMP), and it is particularly preferable that this enzyme is selected from the group consisting of collagenase and gelatinase.

In an even more preferred embodiment, the present invention relates to the implant or gel that comprises at least a compound with magnetic functionality that also comprises a substrate of at least one enzyme involved in the regeneration of the extracellular matrix and a plurality of magnetic particles, as described in this patent application, where the substrate can be selected from the group consisting of collagen, fibrilin, elastin, laminin, osteocalcin, chondroitin sulphate, hyaluronic acid, osteonectin, osteopontin, gelatine and a combination thereof. Preferably, the substrate can be selected from the group consisting of collagen, chondroitin sulphate and hyaluronic acid.

In a particularly preferable embodiment, the implant or gel comprises a compound with magnetic functionality, the magnetic particles are superparamagnetic particles, preferably magnetite nanoparticles, and the substrate can be selected from the group consisting of collagen, chondroitin sulphate and hyaluronic acid. Even more preferably, magnetite nanoparticles are bound by covalent bonds to the substrate.

The present invention also relates to a compound with magnetic functionality that comprises a substrate of at least one enzyme involved in the regeneration of the extracellular matrix and a plurality of magnetic particles, to manufacture an implant or a gel to monitor an enzymatic activity involved in the regeneration of the extracellular matrix. When the product manufactured is an implant, this can be used for monitoring an enzymatic activity involved in the regeneration of the extracellular matrix in a human being or another living being. On the other hand, when the product manufactured is a gel, this can be used to monitor an enzymatic activity involved in the regeneration of the extracellular matrix in vitro.

Preferably, the magnetic particles can be superparamagnetic particles and more preferably magnetite nanoparticles.

In a preferred embodiment, the compound with magnetic functionality used for manufacturing the implant or gel as described also comprises a plurality of magnetic particles bound by covalent bonds to the substrate of at least one enzyme involved in the extracellular matrix regeneration.

In another preferred embodiment, the substrate comprised in the compound with magnetic functionality to manufacture the implant or gel as described in the above paragraph can be selected from the group consisting of protein, modified protein, polypeptide, modified polypeptide, glycoprotein, glycosaminoglycan and a combination thereof.

In an even more preferred embodiment, the present invention relates to the compound with magnetic functionality that comprises a substrate of at least one enzyme in the regeneration of the extracellular matrix and a plurality of magnetic particles as described in this patent application, where the substrate can be selected from the group consisting of collagen, fibrilin, elastin, laminin, osteocalcin, chondroitin sulphate, hyaluronic acid, osteonectin, osteopontin, gelatine and a combination thereof. Preferably, the substrate can be selected from the group consisting of collagen, chondroitin sulphate and hyaluronic acid.

In a particularly preferable embodiment, the magnetic particles are superparamagnetic particles, preferably magnetite nanoparticles, and the substrate can be selected from the group consisting of collagen, chondroitin sulphate and hyaluronic acid. Even more preferably, magnetite nanoparticles are bound by covalent bonds to the substrate. Collagen has a complex structure. Its precursor protein (monomer) is called tropocollagen and is about 300 nanometers in length and 1.4 nm in diameter. Tropocollagen is formed by three polypeptide chains called alpha chains, which in turn are crossed linked to form a triple helix structure. Each $\alpha$ chain is a polypeptide formed by repetition of triplets containing overall glycine amino acids and is very rich in proline and hydroxyproline. Hydroxyproline represents from 10 to 12% of the total of the composition of the collagen residues, this percentage depending on the type of collagen. Each chain has a molecular weight of around 100,000 Da.

There are different types of collagens determined by differences in the composition of their alpha chains:

Type I collagen: abundant in the dermis, bones, tendons and cornea. It occurs as striated fibrils of 20 to 100 nm in diameter, grouped to form greater collagen fibres. Its greater subunits are formed by alpha chains of two types, which differ slightly in their amino acid composition and in their sequence. One of them is designated as the alpha 1 chain and the other as the alpha 2 chain. It is synthesised by fibroblasts, chondroblasts and osteoblasts. Their main function is stretch resistance.

Type II collagen: located particularly in the cartilage, but also in the embryonic cornea and in the notochord, in the pulpar nucleus and in the vitreous humour of the eye. In the cartilage it forms thin fibrils of 10 to 20 nanometres, but in other microenvironments it can form larger fibrils, that cannot be morphologically distinguished from type I collagen.

These are formed by three alpha 2 chains of a single type. It is synthesised by the chondroblast. Its main function is resistance to intermittent pressure.

Type III collagen: abundant in the loose connective tissue, in the walls of blood vessels, the skin dermis and the stroma of several glands. It appears to be a significant component of fibres of 50 nanometres that are traditionally referred to as reticular fibres. It is formed by a single class of alpha3 chain. It is synthesised by the smooth muscle cells, fibroblasts, glia. Its function is to support expandable organs.

Type IV collagen: it is the collagen that forms the basement membrane underlying the epithelia. It is a collagen which is not polymerised in fibrils, but forms a filter of molecules oriented at random, associated with proteoglycans and with the structural proteins laminin and fibronectin. It is synthesised by the epithelial and endothelial cells. Its main function is support and filtration.

Type V collagen: Present in most of the interstitial tissue. It is associated with type I.

Type VI collagen: Present in most of the interstitial tissue. It is the anchorage of the cells in their environment. It is associated with type I.

Type VII collagen: located in the basement membrane.

Type VIII collagen: present in some endothelial cells.

Type IX collagen: located in the mature joint cartilage. It interacts with type II.

Type X collagen: present in hypertrophic and mineralised cartilage.

Type XI collage: located in the cartilage. It interacts with types II and IX.

Type XII: present in tissues subject to high stress, such as tendons and ligaments. It interacts with types I and III.

Type XIII collagen: widely found as a protein associated with the cell membrane. It interacts with types I and III.

Type XIV collagen: non-fibrilar and very similar to type XII, it is present in the foetal epidermis and tendons.

Collagen, in fact, is the substrate of several enzymes of the family of matrix metalloproteases (MMPs) that fulfil several of the main functions in building and renewal of tissues. Therefore, the determination of the collagenolytic activity *in vivo* of said enzymes, often called collagenases, has a great clinical and scientific interest. The collagenolytic activity of some MMPs is derived in part from their tertiary structure depending on calcium and zinc. They are smaller than tropocollagen and have three different regions. One, called propeptidic, has the capacity to bind to collagen chains. Another, known as catalyst, enhances the deployment of the triple collagen helix. The third region is the terminal carboxyl end, and includes four $\beta$ layers that work as helix paddles propelling the enzyme during the dismembering of the three chains in the alpha helix. The $\alpha$ chains released are susceptible of being digested by other enzymes such as pepsin and trypsin.

For the purpose of detecting the activity of these enzymes of interest, enzymes with collagenolytic activity, it is essential and necessary that the collagen used as substrate keeps its triple helix tertiary structure during the immobilisation process of the magnetic particles on the residual surfaces, as well as during the manufacture of the implant or gel comprising the compound with magnetic functionality.

Both chondroitin sulphate and hyaluronic acid are glycosaminoglycans present in the connective tissue and their catabolism is a signal of regeneration of connective tissue. Chondroitin sulphate or chondroitine sulphate are formed by a chain of disaccharides of N-acetylgalactosamide and N-glucuronic acid alternating. This is usually associated with proteins forming aggregates with a high molecular weight, called proteoglycans. A chondroitin chain can be formed by over 100 individual sugars, each of which can be sulphated in diverse positions and number. Chondroitin sulphate provides the cartilage with its mechanical and elastic properties and provides this tissue with much of its resistance to compression. This is digested by a group of enzymes called sulphatases.

Hyaluronic acid shows structural function, like chondroitin sulphates. With a viscous texture, it is present in the synovium, vitreous humour and connective tissue of multiple organisms and is an important glycoprotein in joint homeostasis. In human beings it is mainly concentrated in the joints, cartilages and skin. In an average man of 70 kg in weight there can be a total amount of 15 g of hyaluronic acid in the body and a third of it is broken down and synthesised every day. It is formed by complex carbohydrate chains, specifically about 50,000 disaccharides of N-acetyl glucosamine and glucuronic acid per molecule and derived from the binding of amino sugars and uronic acid. this chain is arranged forming spirals with a mean molecular weight of 2 to 4 million. It shows the property of retaining high amounts of water and adopting an extended form in dissolution, so they are useful for cushioning or lubricating. These properties are achieved thanks to the high number of hydroxyl group and negative charges of this molecule, which allows, by establishing repelling forces, that the carbohydrate chains remain relatively separated from each other.

Hyaluronidase is the enzyme hydrolysing hyaluronic acid in the extracellular matrix.

The present invention also relates to the use of the compound with magnetic functionality as described in this patent application to manufacture an implant or gel for monitoring an enzymatic activity involved in the regeneration of the extracellular matrix. Preferably to monitor regeneration of the extracellular matrix that is part of the connective, bone or organ tissue.

In another preferred embodiment, the compound with magnetic functionality can be used to manufacture an implant or gel to monitor the biological integration of an implant in a human being or another living being. If the compound with magnetic functionality is contained in the gel as described in this patent application, this can be used to monitor biological integration of an implant. On the other hand, if the compound with magnetic functionality is contained in the implant as described in this patent application, this preferably can be used to monitor the biological integration of said implant.

In addition, the present invention also relates to the implant comprising at least a compound with magnetic functionality comprising also a substrate of at least one enzyme involved in the regeneration of the extracellular matrix and a plurality of magnetic particles as described in this patent application, to monitor the regeneration of connective tissue and/or biological integration of an implant. Preferably, the substrate is selected from the group consisting of collagen, chondroitin sulphate and hyaluronic acid, and magnetic particles are superparamagnetic particles. Even more preferably, magnetite nanoparticles are bound by covalent bonds to the substrate.

Furthermore, the present invention also relates to the gel comprising at least one compound with magnetic functionality that in turn comprises a substrate of at least one enzyme involved in the regeneration of the extracellular matrix and a plurality of magnetic particles as described in this patent application, to monitor regeneration of the connective tissue. Preferably, the substrate is selected from the group consisting of collagen, chondroitin sulphate and hyaluronic acid and the magnetic particles are superparamagnetic particles. Even more preferably, magnetite nanoparticles are bound by covalent bonds to the substrate.

The use of an implant comprising at least one compound with magnetic functionality as described in this patent application is not only useful for the evaluation of connective tissue regeneration, but also for the follow-up of therapeutic implants where it is expected that extracellular matrix appears *de novo.*

The implants object of the present invention suitable to be used for monitoring connective tissue regeneration can be gels for injection, patches or dressings to be placed either on the surface of tissue to be regenerated or as grafts. There are several types of connective tissue implants that are routinely used clinically or under development. These implants generally comprise a combination of permanent biocompatible and biodegradable materials. These biodegradable materials usually include components of the extracellular matrix produced by techniques of culture, genetic recombination or extractions. The breakdown, integration and relevant replacement of the components of the implant by endogenous materials produced by the recipient body, is usually related to the invasion of mesenchymal cells and fibroblasts.

As mentioned above, the gels object of this invention are adequate to be used in monitoring connective tissue regeneration *in vitro* and can comprise the same materials mentioned in the above paragraph for the implants.

Examples of such biocompatible, biodegradable material are polylactides, polyglycolides, polycaprolactones, polyanhydrides, polyamides, polyurethanes, polyester amides, poly-ortho esters, polydioxanones, polyacetals, polycetals, polycarbonates, poly-ortho carbonates, polyphosphazenes, polyhydroxybutyrates, polyhydroxyvalerates, polyalkylene oxalates, polyalkylene succinates, poly(malic acid), poly (amino acids), poly (vinyl methyl ether), poly (maleic anhydride), chitin, chitosan and co-polymers, terpolymers or polymers of higher poly-monomer or combinations thereof or mixtures thereof.

Other polymers that can be comprised in the implants or gels of the present invention can comprise one or more recurrent units selected from lineal (siloxanyl)alkyl methacrylates, branched (syloxanyl)alkyl (meth)acrylates, cyclic (syloxanyl)alkyl (meth)acrylates, (meth)acrylates containing silicone, (meth)acrylates containing fluoride, (meth)acrylates containing hydroxyl, (meth)acrylic acid, N-(meth)acryloylpyrrolidone, (meth)acrylamides, aminoalkyl (meth)acrylates, (meth)acrylates containing alkoxy groups, (meth)acrylates containing aromatic groups , glycidyl (meth)acrylate, tetrahydrofurfuryl (meth)acrylate, styrene derivatives containing silicone, styrene derivatives containing fluoride, styrene derivatives and vinyl monomers.

The preferred examples of biocompatible materials include, without excluding other materials, fibrin glue, polyglycolic acid, polylactic acid, polyethylene oxide gel, alginate or calcium alginate gel, poly-(methacrylate of 2-hydroxyethyl), poly-ortho ester, polyanhydride, chitosan, gelatine, agarose and other bioabsorbable and biocompatible materials. In other forms of embodiment, the implant or gel can comprise agar, agarose, gellan gum, Arabic gum, xanthan gum, carrageenan, alginate salts, bentonite, Ficoll, Pluronic polyols, polyvinylpyrrolidone, polyvinyl alcohol, polyethylene glycol, methyl cellulose, hydroxymethyl cellulose, hydroxyethyl cellulose, carboxymethyl cellulose, carboxymethyl chitosan poly-2-hydroxyethyl-methacrylate, polylactic acid, polyglycolic acid, gelatine, a plastic, or a combination of any of them. Hydrogels of poly(methacrylate 2-hydroxyethyl) (pHEMA) are known to those skilled in the art and have been already used as gels of biocompatibles. Polysaccharides are a class of macromolecules of general formula $(CH_2O)_n$ that are also useful as material of the implant or gel in the present invention. Polysaccharides include several compounds of natural origin, for instance, agarose, alginate and chitosan. Agarose is a clear thermoreversible hydrogel made up of polysaccharides, mainly alternating co-polymers of 1,4 linked and 3,6-anhydrous-, Alpha-L-galactose and 1,3 linked. Beta-D-galactose.

A preferred form of preparing the implant or gel is by glycation or cross-linking of elements of the extracellular matrix. In a preferred embodiment of the invention type I, type II collagen or a combination of both is used dissolved in acetic acid at a concentration ranging from 1% to 5%, mixed with ribose or dextrose, stabilised at pH 7.4 and incubated at 4°C in saline phosphate solution and jellified at 37°C. Stabilised suspensions of the compound with magnetic functionality described in this patent application can be added during any of the stages prior to jellifying. In an even more preferred embodiment of the invention, before the jellifying stage chondroitin sulphate or hyaluronic acid are added at a concentration ranging from 1% to 5% and hyaluronic acid can be previously stabilised by cross-linking.

In specific embodiments of the invention, the gel can also comprise a biological or synthetic hydrogel, including alginate, cross-linked alginate, fibrin glue, fibrin clot, poly (N-isopropylacrylamide), agarose, chitin, chitosan, cellulose, polysaccharides, poly(oxyalkylene, a co-polymer of poly(ethylene oxide-poly (propylene oxide), poly (vinyl alcohol), polyacrylate, platelet rich plasma (PRP) clot, platelet poor plasma (PPP) clot or mixtures thereof.

Furthermore, the present invention also relates to the compound with magnetic functionality comprising a substrate or at least one enzyme involved in the regeneration of the extracellular matrix and a plurality of magnetic particles for manufacturing the implant as described in this patent application, to monitor an enzymatic activity involved in the extracellular matrix regeneration in a human being or another living being in an implant. Preferably, to monitor regeneration of connective tissue and/or biological integration of the implant.

In a preferred embodiment, the present invention relates to the compound with magnetic functionality that comprises a substrate selected from the group consisting of collagen, chondroitin sulphate ad hyaluronic acid and a plurality of magnetic particles for monitoring enzymatic activity involved in the regeneration of the extracellular matrix of the implant in a human being or another living being. Preferably, to monitor the regeneration of the connective tissue and/or biological integration of the implant.

The present invention also relates to a method to monitor regeneration of the extracellular matrix, preferably to monitor regeneration of the connective tissue, that comprises using an implant or a gel comprising at least a compound with magnetic functionality that comprises also a substrate of at least one enzyme involved in the regeneration of the extracellular matrix and a plurality of magnetic particles as described in this patent application. Preferably, the magnetic particles are bound by covalent bonds to the substrate.

In a preferred embodiment, the present invention relates to a method to monitor the regeneration of the extracellular matrix, preferably to monitor regeneration of connective tissue, that comprises using an implant or a gel comprising at least a compound with magnetic functionality comprising in turn a substrate selected from the group consisting of collagen, chondroitin sulphate and hyaluronic acid, and a plurality of superparamagnetic particles as described in this patent application.

The present invention also relates to a method to monitor the biological integration of an implant in a human being or another living being as described in this patent application, that comprises using the compound with magnetic functionality that also comprises a substrate of at least one enzyme involved in the regeneration of the extracellular matrix and a plurality of magnetic particles as described in this patent application. Preferably, the magnetic particles are bound by the covalent bonds to the substrate.

In addition, the present invention also relates to a compound with magnetic functionality comprising a substrate of at least one enzyme and a plurality of magnetic particles, characterised in that

i) the magnetic particles do not have any coating or modification of surface,
or
ii) the magnetic particles are functionalised with carboxylic groups.

The compound with magnetic functionality of the present invention allows determining the enzymatic activity of one or more enzymes, as this activity causes a measurable modification in its magnetic properties, in the magnetic properties of one or more of its breakdown products, or in both.

Preferably, the magnetic particles as described in i) or ii) are bound by covalent bonds to the substrate.

In a preferred embodiment, the present invention relates to the compound with magnetic functionality comprising a substrate of at least one enzyme and a plurality of magnetic particles as described in this patent application, where the magnetic particles are superparamagnetic particles. Preferably, the superparamagnetic particles can be ferrite nanoparticles, for instance magnetite, maghemite, cobalt ferrites, silver or magnesium.

In an even more preferred embodiment, the superparamagnetic particles are magnetite nanoparticles, and it is particularly preferable that magnetite nanoparticles have a diameter of less than 15 nm.

The compound with magnetic functionality comprising a plurality of magnetic particles, preferably superparamagnetic particles, functionalised with carboxyl groups and a substrate of at least one enzyme as described in this patent application promotes the binding of magnetic particles to amino groups present in the substrate of at least one enzyme. This compound with magnetic functionality is of particular interest when the substrate comprises a significant number of amino groups, such as chondroitin sulphate and hyaluronic acid, two of the preferred substrates of this invention with monomers that contain amino groups.

In another preferred embodiment, the present invention relates to a compound with magnetic functionality that comprises a substrate of at least one enzyme and a plurality of magnetic particles, where the magnetic particles do not have any coating or modification of surface.

The coatings and modifications of surface reduce the effect of some magnetic particles such as ferrite particles on the transversal (T2) and longitudinal (T1) relaxation times, where the measurement is the basis of the magnetic resonance used currently in medical applications. During agglomeration of the particles, there are two causes with effect on the relaxation times. One is the coupling of the magnetic moments among the magnetic domains of the different particles and the other is the irrigation of the spaces between magnetic particles by water molecules. This irrigation of an aggregate of magnetic particles depends obviously on hydration thereof and is governed by diffusion and uptake of water molecules in the proximities of the same magnetic particles. Both events have an effect on T2, but only the coupling between domains has effect on T1. The existence of surface modifications usually shortens T1 through the same mechanisms of uptake and diffusion of water molecules inside the aggregate that in turn shield the effects of shortening of T1 which are the result of aggregation. During the development of the invention, it has been ultimately seen that the differences in T2 observed in modified collagen to have magnetic functionality before and after treatment with collagenases *in vitro* can be up to 30% higher when using particles with no modifications of surface compared to functionalised particles with surface coatings. In both cases, magnetite particles of 10 nm were used. A more detailed description of the effects on the properties of the magnetic particles isolated as a result of modifications of surface and aggregation thereof is given respectively in Coating thickness of magnetic iron oxide nanoparticles affects R2 relaxivity, LaConte LE, Nitin N, Zurkiya O, Caruntu D, O'Connor CJ, Hu X, Bao G.; J Magn Reson Imaging. 2007 Dec; 26(6): 1634-41 and Water magnetic relaxation in superparamagnetic colloid suspensions: The effect of agglomeration, A. Roch, F. Monky, R.N. Muller, P. Gillis; Magnetic Resonance in Colloids an Interface Science, J. Fraissard, O. Lapina, p. 383-92. The fact that the magnetic particles contained in the compound with magnetic functionality described in this patent application do not have any coating or modification of surface, allows obtaining a greater difference in the times of relaxation measured before the digestion compound with magnetic functionality and after aggregation of the magnetic particles as a result of enzyme digestion.

In another preferred embodiment, the present invention relates to the compound with magnetic functionality that comprises a substrate of at least one enzyme and a plurality of magnetic particles, where i) the magnetic particles do not have any coating or modification of surface, or iii) the magnetic particles are functionalised with carboxylic groups as described in this patent application, where the substrate can be selected from the group consisting of protein, modified protein, polypeptide, modified polypeptide, glycoprotein, glycosaminoglycan and a combination thereof. Preferably, the substrate can be selected from the group consisting of collagen, fibrilin, elastin, laminin, osteocalcin, chondroitin sulphate, hyaluronic acid, osteonectin, osteopontin, gelatine and a combination thereof.

In an even more preferred embodiment, the present invention relates to the compound with magnetic functionality comprising a substrate of at least one enzyme and a plurality of magnetic particles, preferably superparamagnetic particles, where i) the magnetic particles do not have any coating or modification of surface or iii) the magnetic particles are functionalised with carboxylic groups as described in this patent application, where the substrate can be selected from the group consisting of collagen, chondroitin sulphate and hyaluronic acid.

In a particularly preferred embodiment, the present invention relates to the compound with magnetic functionality comprising a substrate of at least one enzyme and a plurality of magnetite nanoparticles that do not have any coating or modification of surface and where the substrate can be selected from the group consisting of collagen, chondroitin sulphate and hyaluronic acid. More preferably, magnetite nanoparticles are bound by covalent bonds to the substrate.

In another preferred embodiment, the present invention relates to a compound with magnetic functionality comprising magnetic particles, preferably superparamagnetic, functionalised with carboxylic groups, where the magnetic particles usually aggregate at physiological pH.

In another preferred embodiment, the present invention relates to a compound with magnetic functionality comprising magnetic particles, preferably superparamagnetic, functionalised with carboxylic groups, where the magnetic groups have modifications only in part of their surface.

In another preferred embodiment, the present invention relates to the compound with magnetic functionality that comprises a plurality of magnetic particles immobilised in a substrate of at least one enzyme, where the magnetic particles do not have any coating or modification of surface and said compound is obtained by the method described below in this patent application.

The present invention relates also to a method for obtaining the compound with magnetic functionality comprising a substrate of at least one enzyme and a plurality of magnetic particles with no coating or modification of surface, as described in this patent application, this method characterised in that it comprises:

a) modifying the substrate of at least one enzyme, and

b) immobilising a plurality of magnetic particles that do not have any coating or modification of surface in the modified compound obtained in stage a). Preferably, the magnetic particles are bound by covalent bonds to the substrate.

In stage a) of modification of the substrate, substances are used that subsequently can bind to the surface of the magnetic particles. This binding reaction is usually promoted by the ionic dissociation of the water in the surface of these particles, and the substances used for the modification of the substrate are highly reactive in aqueous medium.

Therefore, the modification of the substrate is performed preferably under anhydrous conditions and in an organic medium, which allows minimising or preventing the reaction of the substance used for modifying the substrate with water.

Unlike other methods known in the state of the art where the magnetic particles are coated or have a modification of surface, this method allows binding the substrate of the enzyme to magnetic particles that are almost entirely magnetic nucleus, thus obtaining a compound with a magnetic functionality much more sensitive for the determination of enzymatic activity.

Preferably, the present invention relates to the method for obtaining the compound with magnetic functionality comprising immobilising a plurality of magnetic particles without coating or modification of surface, more preferably superparamagnetic particles, in a substrate of at least one enzyme as described in this patent application, where in stage a) the substrate reacts preferably with a compound selected from the group consisting of aminosilanes, aromatic diazonium salts, polymers, polyelectrolytes and bidentate complexes, preferably in an organic medium.

Stage a) of the method for obtaining the compound with magnetic functionality as described in this patent application can be performed using a polymer selected from the group consisting of polyamines, polyethylenes, polyamides and polystyrenes. It can be also used in this stage 1) of modification of substrate a bidentate complex selected from the group consisting of porphyrins and pyrimidines. In addition, in this stage of the method dopamine conjugated with succinic acid in an aqueous medium can be also used.

In an even more preferred embodiment of the invention stage a) of the method can be performed with aminosilanes in an organic medium with a maximum water content of 2%, preferably up to 1%, which allows preventing or minimising contact of aminosilane with water, thus avoiding silanisation reactions or cross-linking between aminosilane molecules.

Aminosilanes belong to the group of alkoxysilanes. Compounds such as mica, glass or surface oxides can be silanised as they contain hydroxyl groups that can be replaced by the alkoxy groups of silanes, leading to the formation of -Si-O-Si covalent bonds. The use of aminosilanes allows binding organic to inorganic elements. Alkoxysilanes are divided into three groups: aminosilanes, which have a secondary or tertiary amino group; glycidoxysilanes with an epoxide; and mercaptosilanes, which have thiols.

In an even more preferred embodiment, the aminosilane used in stage a) of the method for obtaining the compound with magnetic functionality as described in this patent application can be selected from the group consisting of (3-aminopropyl)-triethoxysilane (APTES), (3-aminopropyl)-diethoxy-methylsilane (APDEMS), (3-aminopropyl)-dimethylethoxysilane (APDMES) and (3-aminopropyl)-trimethoxysilane (APTMS).

In a particularly preferred embodiment, the aminosilane used in the stage a) of the method of the present invention is (3-aminopropyl)-triethoxysilane (APTES) in organic medium.

Stage a) of the reaction of aminosilane, preferably APTES, with the substrate occurs in anhydrous organic solvent under adequate conditions to keep the structural integrity of the substrate and enhance dispersion thereof, preferably a protein. Said conditions can change according to the structure of the substrate and are usually function of the polarity and proticity of the solvent. For collagen it is preferred that the solvent is polar to preserve the bonds between prolines and hydroxyprolines. In a preferred embodiment of the invention the solvent used in this stage is acetonitrile. In some embodiments of the invention the structural integrity of the substrate can be only determined by experimental techniques such as spectrography, chromatography or circular dichroism.

The functionalised compound obtained in stage a) of the method of the invention can immobilise particles of iron oxide or other ferrites without coatings or modifications of surface, suspended in organic medium with some amount of dissolved water which enhances the appearance of -OH ions on the surface of said particles.

In another even more preferred embodiment, the method for obtaining the compound with magnetic functionality of said invention comprises an additional stage, prior to stage a) of activation of the amino group of aminosilane, preferably the aminosilane being APTES, with an acid anhydride and an organic base in an organic base with a maximum water content of 2%, preferably up to 1%. Therefore, the amino group of the activated aminosilane as anhydride can react in stage a) of the method of the invention with amino groups present in the substrate.

Preferably, the activation of the amino group of aminosilane can occur in the presence of glutaric anhydride and N,N-diisopropylethylamine, in an organic medium such as chloroform, acetonitrile, pentane, cyclopentane, hexane, cyclohexane, benzene, toluene, 1,4-dioxan, diethyl ether, dichloromethane, tetrahydrofuran, ethyl acetate, acetone, dimethyl formamide or dimethyl sulphoxide.

In another even more preferred embodiment, the method for obtaining the compound with magnetic functionality of the present invention comprises an additional stage, prior to stage a) of activation of the amino group of aminosilane, preferably aminosilane being APTES, with an aldehyde in an organic medium with a maximum water content of 2%, preferably up to 1 %. Therefore, activation of the amino group of aminosilane is also achieved, promoting its reaction in stage a) of the method of the invention with carboxylic groups present in the substrate.

In addition, the present invention also refers to if the method for obtaining the compound with magnetic functionality comprising a plurality of magnetic particles, preferably superparamagnetic particles, functionalised with carboxyl groups and a substrate of at least one enzyme as described in this patent application, where said method comprises the reaction of magnetic particles functionalised with carboxylic groups with the substrate by aldehydes and/or carbodiimides. Similarly, amino groups of the substrate can be modified, preferably a protein, to immobilise them in said particles functionalised with carboxyl groups in its surface such as for instance using dimercaptosuccinic acid.

Some methods for modifying magnetic particles with carboxyl groups are described in Preparation and characterization of carboxyl functionalization of magnetite nanoparticles for oligonucleotide immobilization, Min-Jung Kim, Dae-Hwan Jang, Yong-Ho Choae; Physica Scripta T, 2010, volume 139, page 14024. This method is performed in two stages: a1) the joining between 3-thiophenoacetic acid and magnetic particles, preferably magnetite nanoparticles, occurs at 80°C in acetonitrile as solvent through the interaction with potassium permanganate in the surface of iron oxide; b1; after this first modification, the particles are placed in a dissolution with meso-2,3-diercaptosuccinic acid that is immobilised in the surface thereof.

In a preferred method for performing the invention, functionalised magnetic particles can be obtained with carboxyl groups by modifying their surface with aminosilane previously activated with an aldehyde, in a process comprising two stages:

a2) activating the amino group of an aminosilane with an aldehyde; and
b2) joining the aminosilane activated with the aldehyde according to step a2) to the magnetic particles by interaction between the silane groups and the hydroxyl groups in the iron oxide surface.

Subsequently, these particles functionalised with carboxyl groups can be joined by covalent bonds to the substrate, in particular to the amino groups present in said substrate.

Preferably, the activation of the amino group of the aminosilane can occur in the presence of glutaric acid and N,N-diisopropylethylamine in an organic medium such as chloroform, acetonitrile, pentane, cyclopentane, hexane, cyclohexane, benzene, toluene, 1,4-dioxane, diethyl ether, dichloromethane, tetrahydrofuran, ethyl acetate, acetone, dimethylformamide or dimethyl sulphoxide. In another even more preferred embodiment the activation of the amino group of aminosilane, preferably the aminosilane being APTES, with an aldehyde in an organic medium with a maximum water content of 2%, preferably a maximum of 1%.

The modification of the magnetic particle surface is performed preferably by mixing the magnetic nanoparticles in an ethanol or methanol solution with 0.1% to 1% of activated aminosilane and finally from 0% to 5% of $NH_3$ solution from 10% to 50%. Following this protocol it is expected to obtain the total coverage of the surface of the nanoparticles in a time of 24 h and a coverage that can be partial in shorter times.

In another preferred embodiment, the magnetic particles are only partially functionalised with carboxylic groups. This enhances the aggregation of said particles at physiological pH.

In a preferred embodiment of the invention, step a1) is performed at a time shorter than 30 minutes or at a temperature lower than 80°C. In an even more preferred embodiment of the invention this time ranges from 1 minute to 15 minutes. In another preferred embodiment of the invention this time is shorter than 5 minutes. In another preferred embodiment of the invention, the temperature ranges from 10°C to 60°C.

In another preferred embodiment of the invention step a2) is performed in a shorter time than 24 h. In another even more preferred embodiment of the invention, this time is less than 12 h. In another preferred embodiment of the invention this time ranges from 1 h to 5 h.

In another preferred embodiment of the invention step a2) is performed with a volume concentration of $NH_3$ of less than 10%.

In addition, the present invention also relates to a product comprising at least a compound with magnetic functionality also comprising a substrate of at least one enzyme and a plurality of magnetic particles, where i) the magnetic particles do not have any coating or modification of surface, or ii) the magnetic particles are functionalised with carboxylic groups as described in this patent application, where said product is selected from the group consisting of an implant, a gel, a pharmaceutical composition and a contrast agent.

In a preferred embodiment, the product comprising the compound with magnetic functionality as described in this patent application can be a pharmaceutical composition and also comprise at least one pharmaceutically acceptable excipient.

A preferred administration route of the compound with magnetic functionality as described in this patent application, without excluding other possible methods of administration, is the intravenous administration, preferably local. A detailed list of the possible methods of administration of the possible formulations derived from this invention is given by John M. Walker, Macromolecular drug delivery: methods and protocols, Volume 480, Methods in molecular biology, Springer protocols.

In an even more preferred embodiment, the present invention relates to an encapsulated pharmaceutical composition comprising the compound with magnetic functionality as described in this patent application and at least a pharmaceutically acceptable excipient, and this excipient is adequate to allow releasing the compound with magnetic functionality at the site where the reaction takes place between the modified substrate and at least one of the enzymes reacting with it.

In another even more preferred embodiment, the present invention relates to an encapsulated composition comprising an active ingredient, the compound with magnetic functionality as described in this patent application and at least a pharmaceutically acceptable excipient. Therefore, this encapsulated composition can be used to evaluate the efficacy of administration of said active ingredient.

Particularly preferably, the present invention relates to a pharmaceutical composition that can comprise a compound with magnetic functionality that also comprises a plurality of magnetite nanoparticles, preferably without coatings or modification of surface, immobilised in a substrate selected from the group consisting of collagen, chondroitin sulphate and hyaluronic acid. More preferably, the magnetite nanoparticles are joined through covalent bonds to the substrate.

In another preferred embodiment, the present invention relates to an implant or a gel that comprise at least a compound with magnetic functionality in turn comprising a substrate of least an enzyme and a plurality of magnetic particles, where i) the magnetic particles do not have any coating or modification of surface, or ii) the magnetic particles are functionalised with carboxylic groups as described in this patent application.

Preferably, the implant or gel comprises the compound with magnetic functionality, also comprising a substrate involved in the extracellular matrix regeneration as described above in this patent application and a plurality of magnetic particles that do not have any coating or modification of surface as described in this patent application.

In an even more preferred embodiment, the present invention relates to the implant or gel that comprises the compound with magnetic functionality that also comprises a substrate selected from the group consisting of collagen, chondroitin sulphate and hyaluronic acid and a plurality of magnetic particles, preferably magnetite nanoparticles, that do not have any coating or modification of surface as described in this patent application. More preferably, the magnetite nanoparticles are joined through covalent bonds to the substrate.

In another even more preferred embodiment, the implant or gel comprise the compound with magnetic functionality that comprise in turn a substrate involved in the extracellular matrix as described above in this patent application and a plurality of magnetic particles that do not have any coating or modification of surface and said compound with magnetic functionality is obtained by the method described in this patent application.

In addition, the present invention also relates to the use of the compound with magnetic functionality comprising also a substrate of at least one enzyme and a plurality of magnetic particles, where i) the magnetic particles do not have any coating or modification of surface, or ii) the magnetic particles are functionalised with carboxylic groups as described in this patent application, to manufacture a product as described in this patent application to establish or discriminate the enzymatic activity of one or more enzymes.

In a preferred embodiment, this invention relates to the use of the compound with magnetic functionality that also comprises a substrate of at last an enzyme and a plurality of magnetic particles as described in this patent application for manufacturing a product as described in this patent application to detect a disease or disorder associated with the enzymatic activity of at least one enzyme.

Preferably the disease or disorder can be selected from the group consisting of rheumatic diseases, arthritis, lesion, disease related to the connective tissue, Dupuytren disease, Peyronie disease, disease related to collagen, steatosis, fibrosis, cirrhosis, metastasis, tissue regeneration, cancer, coronary disease, liver disease, metabolic disease, infection and inflammatory disease.

The activity of MMPs plays a role in diseases such as arthritis, rheumatic diseases, conditions related to connective tissue growth as well as Dupuytren or Peyronie diseases, conditions leading to liver fibrosis, cirrhosis or cardiovascular diseases, obstructions of angina of unstable type. A more complete description of the enzymatic activity function of these diseases is given by Woessner JF Jr., in Matrix metalloproteinases and their inhibitors in connective tissue remodelling, FASEB J. 1991 May; 5(8):2145-54 and by Somers KD, Dawson DM in Fibrin deposition in Peyronie's disease plaque J. Urol. 1997 Jan; 157(1):311-5.

Furthermore, the present invention also relates to the use of the compound with magnetic functionality that also comprises a substrate of at least one enzyme and a plurality of magnetic particles, where i) the magnetic particles do not have any coating or modification of surface, or ii) the magnetic particles are functionalised with carboxylic groups as described in this patent application to manufacture the pharmaceutical composition described above to detect a disease or disorder associated with the enzymatic activity of at least one enzyme. Preferably, when the disease or disorder is selected from the list included above.

The follow-up of the activity of MPs deserves special attention in the catabolism of the extracellular matrix, which is related to unstable coronary disease.

The extracellular matrix and a thick fibrous layer provide stability to the atherosclerotic plaque. In general, the unstable plaque has a thin fibrous layer, a high number of inflammatory cells, a large lipid nucleus and can induce the formation of thrombi. Collagen synthesis by the smooth muscle cells is stimulated by growth factors, such as beta-transforming growth factor (β-TGF). The inflammation in the plaques, with the accumulation of macrophages and T cells, leads to the release of MMPs that digest collagen and cause thinning of the fibrous layer. The necrotic lipid nucleus grows as a result of the accumulation of lipids in the extracellular matrix, the death of macrophages loaded with lipids and possibly the accumulation of the erythrocyte membranes after intraplaque bleeding of the vasa vasorum.

Oxygen radicals are generated from various sources, including NADPH oxidase and inflammatory cells, oxidise low-density lipoproteins (LDL) and cause cell necrosis. Repetitive plaque breaking and curing cycles, which can be clinically silent, produce layers in the plaque. Unlike the intracellular proteolytic enzymes located in organelles called lysosomes, MMPs act in the extracellular space and at physiological pH. The superfamily of metalloproteases of the matrix includes MMP1 or interstitial collagenase, an enzyme specialised in the initial division of fibrilar collagens, generally resistant to protease that gives resistance to the fibrous layer of the atheroma. Other members of the family of matrix metalloproteases, such as gelatinases, can catalyse the subdivision of the collagen fragments. Stromelysins can activate other members of the family of MMPs and can break down a wide range of matrix components, including the trunk networks of proteins of the proteoglycan molecules. Stromelysins and one of the gelatinases (gelatinase B or gelatinase of 92 kDa) can also break elastin, a structurally important additional component of the extracellular vascular matrix.

The clinical evidence, supported by molecular models, shows that the risk of significant events in coronary disease, such as obstructions, stroke or acute myocardial infarction are caused by breakage of the plaque due to the MMPS induced by the aforementioned immune excretion of LDL cholesterol, but not by the size of the obstructions.

In another preferred embodiment, the present invention relates to the use of the compound with magnetic functionality also comprising a substrate of at least one enzyme and a plurality of magnetic particles, wherein i) the magnetic particles do not have any coating or modification of surface, or ii) the magnetic particles are functionalised with carboxylic groups as described in this patent application, to manufacture a contrast agent to establish the enzymatic activity of the MMPs in the coronary plaque. Preferably, the substrate may be a protein and this can be selected from the group consisting of collagen, fibrilin and elastin.

The MMPs are also closely related to some liver diseases. Liver fibrosis involves multiple cell and molecular events that lead to deposition of excess extracellular matrix proteins and to a distortion of the normal liver structure. The aetiologies include chronic viral hepatitis, fat liver due to alcohol abuse, non-alcoholic steatosis and drug-related toxicity. Overexpression of MMP is one of the molecular markers that can distinguish hepatic steatosis from fibrosis or cirrhosis. In addition, a type of collagenase (MMP-13) and other MMPs have a transiently increased activity in the first stage of liver fibrosis and a reduced activity in advanced fibrosis.

A net progressive deposition of type I and III collagen has been seen during the development of fibrosis. In addition to the MMP-1, MMP-8 and MMP-13, MMPs in general cannot break type I collagen. MMP-2, MMP-3 and MMP-9 can digest type IV collagen and the enzymatic activity of these MMP decreases progressively with the appearance of cirrhosis. Molecular studies on the expression of mRNA of these enzymes (including those with collagenolytic activity) have shown that they are expressed in the liver, even in cirrhosis, but their activity remains largely under the control by the inhibitors, tissue inhibitor of metalloproteases (TIMP) 1 and 2. The potential for breakdown of the matrix is present, even in advanced cirrhosis, but is contained by the secretion of TIMP.

It is believed that the regression of liver fibrosis is mediated by the reduction in TIMP expression and involves degradation of fibrilar collagen by a combination of MMP-1 and gelatinase A, in addition to interstitial collagenase. Kupffer cells and lypocytes play a key role in the synthesis of MMP and TIMP. Nano- and micro-particles are usually markedly retained by Kupffer cells in the liver.

The administration of collagen modified to have magnetic functionality as described in this patent application is useful to evaluate several liver conditions by measuring the collagenolytic activity of MMPs in the liver. This modified collagen causes a contrast effect caused by the different relaxation times for water protons that are close to the magnetic particles anchored to collagen modified from its distant counterparts. This effect on relaxation is even greater if the magnetic particles are aggregated as a result of enzymatic degradation. This difference is even more significant if the magnetic particles have no surface modifications.

Therefore, the degree of said effect, a greater contrast in the magnetic resonance image, is a signal of expression of MMPs. This increase in the contrast effect of the signal of magnetic resonance can be measured in time by different parameters used in the measurements of magnetic resonance image as intensity of the signal, T1 or T2 weighted images, calculation of diffusion parameters, images of saturation or a combination thereof, without excluding other MRI parameters not listed in this application. A short description of some of these parameters is given as follows. A more complete description of the MRI parameters and their principles is provided by Mitchell et al. in the book "MRI Principles".

When collagen modified to have magnetic functionality by immobilisation of magnetic particles as described above is administered to the liver, as compared to a normal liver, in the final stage of steatosis or hepatosteatosis and principles of fibrosis a greater contrast effect can be seen by analytical techniques such as magnetic resonance. However, in the stages following fibrosis or cirrhosis this contrast effect is more moderate.

The expression of MMPs is also significantly higher in patients with liver metastasis. In cancer, the balance between production and activation of MMPs and their inhibition by TIMPs is a key factor in invasion and metastasis. MMPs are synthesised in the tissues and are filtered by the blood flow. It has been seen that in colorectal, breast, prostate and bladder cancer most patients with severe disease have increased their plasma levels of gelatinase B.

In particular, in patients with advanced colorectal cancer a relationship has been seen between high levels of gelatinase B or complex TIMP associated with a shorter survival. Therefore, it is within the scope of the invention to use compounds with magnetic functionality as substrates to evaluate the expression and/or enzymatic activity and their consequences in cancer and, in general, the activity of MMP and other enzymes in organs, tissues or other body compartments.

In another preferred embodiment, the present invention relates to the use of the compound with magnetic functionality comprising also a substrate of at least one enzyme and a plurality of magnetic particles, where i) the magnetic particles do not have any coating or modification of surface, or ii) the magnetic particles are functionalised with carboxylic groups as described in this patent application, to manufacture an implant or gel to monitor an enzymatic activity involved in the regeneration of the extracellular matrix, preferably in cartilage regeneration.

In another even more preferred embodiment, the present invention relates to the use of the compound with magnetic functionality comprising also a substrate of at least one enzyme and a plurality of magnetic particles, where i) the magnetic particles do not have any coating or modification of surface, or ii) the magnetic particles are functionalised with carboxylic groups as described in this patent application, to manufacture an implant to monitor the biological integration of the implant in a human or another living being.

Preferably, the compound with magnetic functionality described in this patent application comprising magnetite nanoparticles functionalised with carboxylic groups and immobilised in chondroitin sulphate, is added to an aqueous mixture that comprises an additional amount of chondroitin sulphate or another glycosaminoglycan or glycoprotein adequate to manufacture a cartilage implant. Therefore, a gel is obtained that can be added to a cartilage implant and act as reporter of hydrolase activity of chondroitin sulphate or sulphatase, which play a role in cartilage regeneration. In addition, this gel can be also used as reporter of activity of hydrolases of chondroitin sulphate or sulphatase in vitro.

Similarly and alternatively to chondroitin sulphate, in another embodiment of the invention hyaluronic acid can be also used.

When the compound with magnetic functionality that comprises a plurality of magnetic particles immobilised in a substrate of at least one enzyme as described in this patent application is administered to a living being, preferably a human being, this compound allows determining the enzymatic activity of one or more enzymes reacting with said substrate. This determination is performed measuring the variation caused by this enzyme reaction in a parameter associated with the magnetic properties of the compound with magnetic functionality, of at least one of its reaction products, or a combination of both.

In another preferred embodiment, the present invention relates to the use of:

- a compound with magnetic functionality that also comprises a substrate of at least one enzyme involved in the cell regeneration method and a plurality of magnetic particles to manufacture an implant or gel as described in this patent application; or

- a compound with magnetic functionality comprising also a substrate of at least one enzyme and a plurality of magnetic particles, where i) the magnetic particles do not have any coating or modification of surface, or ii) the magnetic particles are functionalised with carboxylic groups as described in this patent application;

where the enzyme reaction causes a change at least partial from superparamagnetism to paramagnetism.

There are several analytical techniques that can be used to establish the effects of the enzymatic activity using the compound with magnetic functionality described in this patent application. Therefore, in another preferred embodiment, the present invention relates to the use of the compound with magnetic functionality as described in this patent application, to establish the variation in the magnetic property caused by the enzyme reaction by a technique selected from the group consisting of magnetic resonance imaging (MRI), electronic paramagnetic resonance (EPR), magnetometry including vibrational magnetometry (MVS), and Superconducting Quantum Interference Devices (SQUID), Mössbauer spectrometry, X-ray magnetic circular dichroism (XMCD), ferromagnetic resonance (FMR), magnetic optic Kerr effect (MOKE), Brillouin light scattering (BLS), electromagnetic inductance, atomic force microscopy, magnetorelaxometry, and a combination thereof.

Some of these techniques can be used to establish *in vitro* or *ex vivo* the existence of enzymatic activity by establishing the spectra and curves of samples previous dried or freeze-dried containing substrates modified to have magnetic functionality and observing changes in said spectra or curves based on the aggregation status of the magnetic particles present in substrates modified to have magnetic functionality before and after exposure to a biological agent. A brief description thereof is given below.

EPR spectroscopy measures the energy differences between the states caused by the splitting generated when placing a decoupled electron system in an external magnetic field (Zeeman field). The difference of energies between two states will be the gμBHZ Form, where g is the Landé factor, μB is the Bohr magnetic field and H the applied magnetic field. The so-called resonance condition for obtaining a resonance peak in the spectrum is:

$$h\nu = g_j \mu_B H_Z$$

where the field $H_Z$ is the resonance field. This expression can be used to obtain the Lande factor g. This factor g can eventually be very useful when identifying the sample, but it is not unique and will depend on the frequency selected for irradiation. Other data provided by the spectra, such as intensities, number of lines, distance between them, their shape and width, amongst others are also other sources of information very useful to identify and characterise the sample. Therefore, the electrons of magnetic particles, particularly those closest to the surfaces thereof, will produce different resonance signals based on the aggregation status of the magnetic particles which are translated into changes in the spectrum.

MVS and SQUIDs measure directly the magnetic fields based on the magnetisation of a sample and determine variation curves thereof.

Mössbauer spectroscopy is a spectroscopic technique based on emission and resonant absorption of gamma rays in solids. It is similar to magnetic resonance imaging (MRI) spectroscopy in that it probes nuclear transitions and is thus sensitive to the similar electron-nucleus interactions as cause of the chemical NMR shifting. The emitting element to characterise compositions with $Fe^{57}$ is usually $Co^{57}$. Typically, there are three types of nuclear interactions that are seen, isomer shifting, division of the quadrupole and hyperfine division. The hyperfine splitting (Zeeman splitting) is a result of the interaction between the nucleus and any surrounding magnetic field. Typically, this splits the single peak into six non-degenerate peaks. The hyperfine splitting is usually measured as the distance between the outermost areas of these six peaks. The hyperfine splitting is particularly important in Mössbauer spectroscopy of compounds containing iron, which are frequently ferromagnetic or antiferromagnetic, resulting in strong internal magnetic fields. In addition to the identification, the relative intensities of the various peaks show the relative concentrations of the compounds in the sample and can be used for the semiquantitative analysis. Also, because the ferromagnetic phenomena are dependent on size, the spectra can provide information on the size of the crystalline and granular structures of a material containing iron.

The X-ray magnetic circular dichroism (XMCD) is the difference between two X-ray absorption spectra, taken applying a magnetic field, one with right circularly polarised light and another to the left (LCP). The greatest advantage of this technique is its sensitivity to each element, which allows characterising magnetically specific elements and finding magnetic moments of minority elements or diluted in a matrix. As the measure of X-ray absorption is performed for energies corresponding to the edge of absorption of a specific element, only the particular contribution of this element is observed. This characteristic is particularly interesting when handling nanoparticles, as it allows separating the contribution of other elements or possible impurities to total magnetisation. The effect is based on the fact that X-ray absorption by the material will be dependent on light polarisation and the spin and orbital moments of the electrons. Measuring the sample absorption for both circular polarisations (left or right) and subtracting both spectra, the XMCD signal is obtained. It is then possible to calculate the magnetic moment of an electron by means of the so-called sum rules. This technique allows also separating the spin and orbital components to the magnetic moment.

In the ferromagnetic resonance technique (FMR) a magnetic field is applied in parallel to the plan of a film containing the sample. The sample is placed on a goniometer which allows turning the plan thereof from the direction of the field. The resonance field, HR, and width of line peak to peak, ΔH, can be obtained as functions of the angle of the field in the plan, of the temperature and the thickness of the film are obtained.

When a polarised light ray is reflected on the surface of a magnetised material, the polarisation plan can rotate slightly. This effect is known as magneto-optic Kerr effect or MOKE, which is widely used in the characterisation of thin films of ferromagnetic materials . Similarly to FMR the sample is placed on a goniometer which allows turning the plan thereof from the direction of the field, but, unlike it, an estimation of the area of a curve of hysteresis is obtained from the measurement of the refraction coefficients for the different orientation angles of the sample.

Brillouin scattering is an optical phenomenon that can be seen when there are magnetic irregularities in a film or medium that is crossed by an incident beam.

In another preferred embodiment of the invention, these analytical techniques allow detecting enzymatic activity *in vivo* and can be selected from the group consisting of magnetometry and magnetic resonance imaging. In an even more preferred embodiment of the invention, this analytical technique is magnetic resonance imaging.

In another preferred embodiment, the present invention relates to the use of the compound with magnetic functionality as described in this patent application, where the enzyme reaction causes a variation in the magnetic properties, preferably at least a partial change of superparamagnetism to paramagnetism, detectable by determination of a parameter selected from the group consisting of intensity of signal, longitudinal relaxation time (T1), transversal relaxation time (T2), diffusion parameter, response signal to a first series of pulses, saturation signal I and a combination thereof. Preferably, the variation in the magnetic properties caused by the enzyme reaction can be measured by a parameter selected from the group consisting of longitudinal relaxation time (T1), transversal relaxation time (T2) and ratio of T1 to T2. Preferably, said variation can be determined by the ratio of T1 to T2.

Preferably, the variation in the magnetic properties caused by the enzyme reaction can be determined analysing said parameter before and after the enzyme reaction occurs as described in this patent application. In particular, the parameter is analysed before and after the reaction takes place between the compound with magnetic properties object of the invention comprising a substrate of one or more enzymes, and one or more of these enzymes.

The measuring methods based on magnetic resonance, for instance, magnetic resonance imaging or electronic paramagnetic resonance (EPR) are indirect methods adequate to measure variations in the magnetic properties of the compounds with magnetic functionality, of at least one of its breakdown products or a combination thereof. In the case of the magnetic resonance imaging methods the relaxation times of the response to the effect of the application of radiofrequency (RF) perpendicular to an intense magnetic field are measured. These relaxation times are called T1 and T2 and represent the characteristic times of relaxation of water protons as a result of the disappearance of a magnetic field in the plan longitudinal to the RF applied and the reappearance of the force of the magnetic field in the perpendicular to the plane of the RF applied, respectively, as a result of the elimination of said RFs. As compared to the diamagnetic materials as most tissues and biological fluids, T1 and T2 are significantly shorter in the presence of ferromagnetic, paramagnetic and superparamagnetic materials. For a long time it is also known that the addition of paramagnetic solutes causes a reduction in the measurement of parameters T1 of water hydrogen protons.

Magnetic resonance, as used in clinical applications, is based on the balance between the extremely small magnetic moment of a proton and the very high number of protons present in the biological tissue, which leads to a measurable effect in the presence of large magnetic fields. For $B_0$ parallel to axis z these relaxation signals are:

$$m_z = m\left(1-e^{-t/T_1}\right)$$

and

$$m_{x,y} = m\sin(\omega_0 t + \varphi)e^{-t/T_2}$$

Where $m_z$ is the magnetisation along the axis z, $m_{xy}$ the magnetisation perpendicular to the axis z T1 T2 are the longitudinal and transversal relaxation times, respectively, $\omega_0$ is the frequency of precession and $\varphi$ is a phase constant. The longitudinal relaxation reflects a loss of energy, such as hot, from the system to its surrounding network, and is particularly a measure of the bipolar coupling of the proton moments to their environment. Relaxation in the plane xy is relatively fast and is promoted by the loss of coherence of phase in the precession of protons due to their magnetic interactions with each other and with other fluctuations of the magnetic moments in the tissue. A lag can be also affected by local non-homogeneities in the scope of longitudinal application, giving rise to the substitution of T2 in the formula of second of the shorter relaxation times, T2*:

$$\frac{1}{T_2^*} = \frac{1}{T_2} + \gamma \frac{\Delta B_0}{2}$$

Where $\Delta B_0$ is the variation in the field occurring either through distortions in the homogeneity of the field applied or local variations in the magnetic susceptibility of the system. This $\Delta B_0$ variation can be exactly caused by magnetic particles, which cause a significant predominance on the possible local factors of non-homogeneity of the B0 field. For some embodiments of the invention these differences resulting in $\Delta B_0$ caused by the changes in the distribution or aggregation state of magnetic particles which are first immobilised on substrates or other macromolecules of interest, the cause of the signal detected as a consequence of breakdown or metabolic use of said substrates or other macromolecules of interest.

In another preferred embodiment, the present invention relates to the use of the compound with magnetic functionality as described in this patent application, where the variation in a magnetic property indicates at least a physical phenomenon selected from the group consisting of:

- aggregation of magnetic particles,
- aggregation of compounds with magnetic functionality or at least one of the reaction products thereof,
- change in the size of the magnetic particles, and
- change in the particle size of at least one selected from the group consisting of the compound with magnetic functionality object of the present invention, at least one of the products of reaction thereof and a recombination thereof.

It must be highlighted that the effect of aligning the magnetic moments of magnetic particles can change markedly as a result or their grouping or distribution. It can be understood from two factors 1) the effects or thermal or kinetic agitation, as explained above, and 2) the average time and energy necessary for responding to an external magnetic field applied as explained below.

A special case of interest is that where the magnetic particles are superparamagnetic and tend to be aggregated as a result of the variation of such conformations. Then the resulting magnetic aggregate, that must not be necessarily superparamagnetic, has magnetic properties that differ measurably from the magnetic properties of the original magnetic particles. Therefore, when determining variations in magnetic properties, a signal of an enzyme effect can be detected. In the case of magnetite nanoparticles of less than 15 nm in diameter, they behave as superparamagnetic when they are suspended in aqueous solutions at biological temperatures, but their behaviour becomes paramagnetic if aggregated forming piles of greater diameter.

Figure 1 shows the effect of the size and aggregation of the magnetite superparamagnetic and paramagnetic nanoparticles upon measuring the inverse of T2, also called magnetic relativity R2. It can be seen that the superparamagnetic nanoparticles, once aggregated, behave more similarly to paramagnetic particles.

If a magnetic material is placed on a magnetic field of intensity H, the individual atomic moments in the material contribute in response aligning in the direction thereof. This is called magnetic induction:

$$B = \mu_0 (H + M)$$

Where $\mu_0$ is the magnetic permeability of the medium, and magnetisation M = m / V is the magnetic moment per volume unit, wherein m is the magnetic moment in a material with volume V. all materials have a magnetic response based on their atomic structure and temperature. They can be appropriately classified in terms of volumetric magnetic susceptibility, $\chi$, where $M = \chi H$ describes induced magnetisation in a material by H. In the IS units $\chi$ is adimensional and M and H are expressed in $Am^{-1}$.

The susceptibility of the materials not only depends on temperature, but also on the intensity of the magnetic field (H), giving rise to the characteristic sigmoid shape of the M-H curve, with M approaching a saturation value at high H values. On the other hand, in ferromagnetic and ferrimagnetic materials often a curve of hysteresis appears that shows irreversibility in the magnetisation process related to the spin of the magnetic domains in the walls of impurities or limits of granulation inside the material, as well as for intrinsic effects, such as magnetic anisotropy of the crystalline network. This leads to the fact that the M-H curves generate hysteresis loop. The shape of these cycles is determined in part by the particle size: in large particles (of the order of micrometres or more) is a fundamental state of several domains which leads to a cycle of narrow hysteresis, as it requires relatively low field energy to make that the walls of the domains move. In smaller particles the baseline state of the single domain determines the existence from hysteresis. In even smaller sizes (or the order of tenths of nanometres or less) superparamagnetism is seen where the magnetic moment of the particle is free to fluctuate in response to thermal energy, while the relative individual atomic moment keep their orderly condition. This originates sigmoid M-H curves, lacking hysteresis. Even for superparamagnetic particles arranged in bulk, their magnetic moments interact affecting the M-H curves (in some cases even hysteresis can be seen) as well as the relaxation times, giving rise to other magnetic and optical effects.

This effect can be seen for instance by magnetic vibrational spectrometry (MVS). Figure 2 represents the M-H curve of type I collagen functionalising with superparamagnetic nanoparticles of magnetite with a diameter of 10 nm in 10 ml in saline suspension immobilised in agarose.

Figure 3 represents the H-M curve of a similar suspension immobilised in agarose after incubation with type I collagenase for 12 hours at 37°C. Figure 3 shows a slightly open curve; however, the H-M curve of Figure 2 does not show hysteresis. This magnetic behaviour is due to the interaction between the magnetic moments of the nanoparticles overall after aggregation thereof as a result of the collagenolytic activity of collagenase. When collagenase hydrolyses the modified collagen, the alpha chains separate and the anchored magnetic nanoparticles tend to aggregate as a result of the thermal movement inducing magnetic coupling between the magnetic domains of the same magnetic as they aggregate.

A preferred technique for performing the measurement is magnetic resonance, where accumulation or dispersion of magnetic particles can be measured by the changes in the relaxation times and, therefore, also in the effect of contrast of such magnetic particles. In some preferred embodiments of the invention, collagen can be provided either as isolated alpha chains or fragments of protocollagen both naturally occurring or synthetic following a similar process to immobilise magnetic particles that can be performed in $\alpha$ chains or monomers isolated. $\alpha$ chains may be induced to adopt a triple-helix structure, such as the suspension or dissolution in them of an acetic medium treatment such as pepsin, washing, filtration, washing, filtration and collection of precipitates. Figure 4 shows a scheme of a modified collagen tripeptide (A), functionalised with magnetite nanoparticles (B). The drawing is not in scale and the distribution of the magnetite nanoparticles is not necessarily consistent with a real distribution. Figure 5 shows the effect of the collagenolytic activity on the distribution of magnetic nanoparticles first immobilised in collagen after the collagen is digested by collagenases.

## BRIEF DESCRIPTION OF THE FIGURES

Figure 1 shows the evolution of the nuclear magnetic relaxation with particle size or aggregation state, according to an embodiment of the invention. The magnetic relaxation is given in $ms^{-1}$, at different concentrations, measured in mM of iron per litre, produced by three different particle suspensions: 10 nm dispersed by sonication (triangles), 50 nm dispersed by sonication (squares) and 10 nm aggregated (rhombuses).

Figure 2 shows the M-H magnetisation curve of type IV collagen modified with superparamagnetic nanoparticles of magnetite with a diameter of 10 nm in 10 ml of saline suspension and immobilised in agarose, according to an embodiment of the invention. The horizontal axis represents the H field applied and the vertical axis the M magnetisation measured in amperes per turn per meter.

Figure 3 represents the H-M magnetisation curve of a suspension prepared as indicated for Figure 2 immobilised in agarose after incubation with collagenase for 12 hours at 37°C, according to an embodiment of the invention. The horizontal axis represents the H field applied and the vertical axis the magnetisation M, both measured in amperes per turn per meter.

Figure 4 shows a scheme of a triple-helix region of modified collagen A), functionalised with magnetite nanoparticles (B), according to an embodiment of the invention.

Figure 5 shows the effect of the collagenolytic activity on the distribution of magnetic nanoparticles first immobilised in collagen and after said collagen is digested by collagenases, according to an embodiment of the invention.

Figure 6 shows the comparison of maps of T2 seen through the axial axis of two sample tubes (C) and (D) where 0.5 mg of collagen modified to have magnetic functionality by immobilisation of magnetite particles of 10 nm in diameter with no changes of surface in 10% of its lysines. In both tubes said modified collagen is immobilised in a suspension of 1 ml of 10% agarose in a solution of 10 mM of calcium chloride and 400 mM of sodium chloride at pH 7.4 adding 1 mg of collagenase only in tube C. Tube D is a control. Both tubes were placed in incubation for 1 h at 37°C. The average of the maps of T2 of both tubes is 168 ms for (C) and 150 ms for (D), according to an embodiment of the invention.

Figure 7 shows the comparison of maps of T2 seen through the axial axis of two sample tubes (E) and (F), where 0.5 mg are of unmodified collagen as control. In both tubes, said collagen is immobilised in a 10% suspension of agarose in 1 ml of solution of 10 mM of calcium chloride and 400 mM of sodium chloride at pH 75 after adding 1 mg of collagenase only in the tube (E). Tube (F) is a control. Both tubes were placed in incubation for 1 h at 37°C. The average of the T2 maps of both tubes is 191 ms for (E) and 189 ms for (F), and these relaxation times are different from those seen in the modified collagen of Figure 6, according to an embodiment of the invention. The differences of the relaxation times in the T2 maps is difficult to notice visually as shown in this figure.

Figure 8 shows schematically the activation of (3-aminopropyl)-triethoxysilane (APTES) (G) with glutaric anhydride (H) mediated by N,N-diisopropylethylamine (DIEA) (I), to enhance joining to an amino group that belongs to a collagen lysine, according to an embodiment of the invention.

Figure 9 shows schematically the modification of an amino group that belongs to a collagen lysine (K) by linking modified APTES (J) as shown in Figure 8 mediated by carbodiimide diisopropyl (DIPCD) (L) to allow anchorage of a magnetite particle with no modifications of surface in the modified collagen (M) according to an embodiment of the invention.

Figure 10 shows schematically the joining of an amino group that belongs to a polylysine monomer (N) with activated APTES (J) as shown in Figure 8, mediated by DIPCD (L) to produce polylysine modified by APTES (N) to enhance the anchorage to a nanoparticle, according to an embodiment of the invention.

Figure 11 shows the modification of a magnetite particle with no modifications of surface (O), with at least a modified polylysine (N) as that shown in Figure 10 to produce a magnetic particle modified with a polylysine (R), which can be linked by the intervention of carbodiimides to an endogenous collagen lysine, according to an embodiment of the invention.

Figure 12 shows the modification of the n-acetyl amine group of a chondroitin sulphate (S) monomer by linking N-(3-dimethylaminopropyl)-N'-ethylcarbodiimide hydrochloride (EDC) (T) to allow anchorage of a magnetite particle modified in its surface with dimercaptosuccinic acid (U), according to an embodiment of the invention.

EXAMPLES

The following examples are given by way of illustration and it is not intended that they are limitative of this invention.

EXAMPLE 1 - PREPARATION OF A COMPOUND WITH MAGNETIC FUNCTIONALITY BY MODIFICATIONS OF COLLAGEN WITH MAGNETIC NANOPARTICLES WITH NO SURFACE MODIFICATIONS

Collagen from bovine tendon of commercial type has a triple-helix structure and proven properties as substrate of collagenolytic activity. The selection of collagen fragments with a triple-helix structure can be performed through treatment with pepsin and subsequent filtering. The efficiency of the latter process can be confirmed by circular dichroism.

To modify this type of collagen first APTES is bound to its lysines. APTES is activated with glutaric anhydride to form a complex. This reaction is performed with N, N-diisopropylethylamine (DIEA). APTES, glutaric anhydride and IDEA, are dissolved in acetonitrile at a ratio of 1:1:3 and placed under agitation for 24 hours in a dry atmosphere. The collagen is dissolved in acid medium with a diffractive such as mannitol and subject to freeze-drying. The freeze-dried collagen is suspended and homogenised in acetonitrile, adding the resulting mixture to the activated APTES at a 28:1 ratio, adding diisopropyl carbodiimide (DIPCD) at a 1:1 ratio and mixing in a dry atmosphere for other 48 hours. Acetonitrile is replaced by ethanol by evaporation and the excess APTES, compounds other than collagen, are removed by repeated centrifugation and the preparation is suspended again in ethanol. Magnetite particles of less than 15 nm in diameter in suspension in a basic medium, generally aqueous, are mixed in ethanol at an ethanol/water ratio of at least 98:1 and subject to sonication. They are added to the mixture and subject again to mixing in dry atmosphere for other 48 hours. Ethanol is replaced by water evaporation stabilised at pH 3.5 with acetic acid and subject to stirring for 8 hours at 4°C. The resulting suspension is ultracentrifuged for 14 hours on an iodoxinadol bed. The upper bed is removed and subject to dialysis for 24 hours in membrane tubes with pores of 30 KD at 4°C. Once the preparation has been dialysed, mannitol or another alternative surfactant can be added and the preparation is freeze-dried.

The excess can be removed through columns, centrifugation gradient or magnetic separation, dialysed and freeze-dried. The resulting formulation is useful for the detection of the collagenolytic activity *in vivo*, *in vitro* or *ex vivo.* Due to the triple-helix structure of collagen, once the modified collagen for magnetic functionality is broken down by a collagenase the magnetic particles originally immobilised in it will tend to aggregate or disperse based on their baseline distribution along the collagen chains.

Immobilising 3 mg of preparation of collagen modified to have magnetic functionality as described in this example in a suspension 1 mL of 10% agarose in solution of 10 mM of calcium chloride and 400 mM of sodium chloride at pH 7.5 and after adding 1 mg of collagenase a relaxation time T2 of 170 ms was seen, measured using a MRI equipment of 4.7 T. In the absence of collagenase the T2 value seen was 150 ms. When unmodified collagen was used the relaxation times were 191 ms and 189 ms for samples with or without collagenase, respectively.

EXAMPLE 2 - PREPARATION OF A COMPOUND WITH MAGNETIC FUNCTIONALITY IN THE MANUFACTURE OF AN IMPLANT

In this first example APTES is bound to polylysine to then anchor the modified particles. This modified polylysine can be bound to collagen by cross-linking mediated by the lysyl oxidase enzyme. The polylysine is activated with glutaric anhydride prior to binding to APTES. This activation reaction is performed dissolving APTES, glutaric anhydride and N, N-diisopropylethylamine (DIEA) in acetonitrile at a ratio of 1:1:3. Subsequently, stir for 24 hours in a dry environment. Suspend polylysine in acetonitrile, add the resulting mixture to the activated APTES at a 1:1 ratio, together with DIPCD at a 1:1 ratio and mix in a dry environment for other 48 hours. Acetonitrile is replaced by ethanol by evaporation and the excess APTES and other compounds different from modified polylysine are removed by repeated centrifugations and re-suspension in ethanol. Magnetite particles of less than 15 mm in diameter in suspension in an aqueous basic medium are mixed with ethanol at an ethanol/water ratio of at least 98:1. Sonicate, add to the mixture of modified polylysine modified in ethanol previously obtained and mix again in a dry environment for other 48 hours. Ethanol is replaced by water by evaporation, it is stabilised at a basic pH to keep the stability of the modified particles.

A solution of 1.5 mg/ml of type I collagen in 0.1 acetic acid stabilised at pH 7.4 with 0.4 M NaOH in 10X D-PBS and 250 mM HEPES, add 0.02 mg/ml of lysyl oxidase in 25 mM Tris-HCl, pH 8.0, 100 mM NaCl, 0.05% Tween-20, 20% glycerol and 3 nM DTT, the magnetic particles functionalised with polylysines at a final concentration of 1 mM, hyaluronic acid at a final concentration smaller than 1%, stabilise again the mixture at pH 7.4 and allow to incubate for 72 h at 4°C. After incubation perform washing and sonication at 4°C with a NaOH solution at pH 8. Finally, place for incubation the gel at 37°C for 45 minutes.

The magnetic particles modified with polylysines are joined to the collagen by the transformation of the lysines into alysines by the enzyme lysyl oxidase which also enhances bonds alysine - alysine. The resulting gel is semisolid and once implanted it can replace connective tissue. The implantation of said implant involves invasion to it by fibroblasts which secrete collagenases, also breaking down the collagen of the implant and replacing it by collagen with a morphology similar to that of the surrounding tissue. The previous breakdown of collagens causes aggregation of magnetic particles that can be detected by maps of weighted magnetic resonance imaging.

EXAMPLE 3 - MODIFICATION OF CHONDROITIN SULPHATE CHAINS WITH MAGNETIC NANOPARTICLES FUNCTIONALISED WITH CARBOXYL GROUPS TO BE INCLUDED IN CARTILAGE IMPLANTS

Mix 300 microlitres of APTES, 414 microlitres of DIEA and 16 mg of glutaric anhydride in 200 millilitres of acetonitrile for 24 hours. Add 250 ml of ethanol with a min purity 99%, 2 ml of magnetite particles of 10 nm suspended of acid solution with an approximate iron concentration of 16 mg/ml and 10 ml of solution of $NH_3$ at 30%. Allow the mixture to stir for 3 hours and replace ethanol by acetic acid at pH 2 with two washings. Thus magnetic nanoparticles with a surface partially modified with carboxyl groups are obtained.

Salts of chondroitin sulphate are dissolved in a 10 mM solution of 4-(2-hydroxyethyl)-1-piperazinotanosulphonic) acid HEPES. Add the magnetite nanoparticles with their surface partially functionalised obtained previously, with an iron concentration about 1 molar from the monomers of chondroitin sulphate, together with N-(3-dimethlyaminopropyl)-N'-ethylcarbodiimide hydrochloride (EDC) in an excess of 1.21:1 also from the monomers of chondroitin sulphate. The resulting suspension is stabilised at pH 7.4, allow to stir at 4°C for 24 h and remove the excess by filtration or dialysis. Nanoparticles functionalised with chondroitin sulphate are added to aqueous mixtures of additional chondroitin sulphate, other glycosaminoglycans or glycoproteins used in cartilage implants. The mixture is stirred to form a gel. Said gel can be included in cartilage implants and act as reporter of the activity of hydrolases of chondroitin sulphate or sulphatase, which fulfil a role in cartilage regeneration.

EXAMPLE 4. MANUFACTURE OF A GEL COMPRISING A COLLAGEN WITH MAGNETIC FUNCTIONALITY

A solution of 1.5 mg/ml of type I collagen in 0.1% acetic acid is stabilised at pH 7.4 with 0.4 M NaOH in 10X D-PBS and 250 mM HEPES, ribose is added to reach 250 mM, a suspension of collagen with magnetic functionality at a concentration of 1.5 mg/ml, hyaluronic acid at a final concentration lower than 1%, the mixture is stabilised again at pH 7.4, and allow to incubate at 4°C for 72 h. Subsequently, washing and sonication are performed at 4°C with a solution of NaOH at pH 8. Finally, the gel is subject to incubation at 37°C for 45 minutes.

The resulting gel is semisolid and can be used for assays in vitro or form an implant that, once implanted, can replace connective tissue. The inclusion of hyaluronic acid enhances invasion thereof by fibroblasts which secrete collagenases and also degrade the collagen of the implant and replace it by collagen with a morphology similar to the surrounding tissue. The previous collagen degradation causes aggregation of magnetic particles which can be detected by weighted magnetic resonance imaging maps.

EXAMPLE 5: MANUFACTURE OF AN IMPLANT COMPRISING HYALURONIC ACID WITH MAGNETIC FUNCTIONALITY

Following the conditions described in example 4, collagen with magnetic functionality is replaced by a 2% solution of hyaluronic acid with magnetic functionality stabilised at pH 7.4. The implant obtained allows to detect degradation of said implant by the activity of hyaluronidase.

Several types of implants can be also combined concentrically leading to jellifying a new incubation at 4°C on an implant previously prepared, but with a compound with different functionality. Therefore in the same implant more than one enzymatic activity can be detected, depending of the region where it occurs.

EXAMPLE 5: MANUFACTURE OF AN INJECTION IMPLANT COMPRISING HYALURONIC ACID WITH MAGNETIC FUNCTIONALITY

A suspension of 4 mg/ml of type I collagen with magnetic functionality in 0.1% acetic acid is mixed with a solution of hyaluronic acid at a 3% concentration also in 0.1% acetic acid. The mixture is stabilised at pH 7.4 with 0.4 M NaOH in 10X D-PBS and 250 mM HEPES at 4°C and is led to a final concentration of 1 mg/ml of type I collagen with magnetic functionality and 1% of hyaluronic acid at pH 7.4.

The resulting gel, once implanted, can give elasticity and replace connective tissue once injected. The presence of hyaluronic acid enhances invasion thereof by fibroblasts which secrete collagenases and also degrade the collagen of the implant and replace it by its own collagen. The previous collagen degradation causes aggregation of magnetic particles that can be detected by weighted magnetic resonance imaging maps.

EXAMPLE 6: PREPARATION OF A COMPOUND WITH MAGNETIC FUNCTIONALITY BY MODIFICATIONS WITH MAGNETIC NANOPARTICLES FUNCTIONALISED WITH CARBOXYL GROUPS

100 microlitres of magnetite particles of 10 nm modified with carboxyl groups at a concentration ranging from 10 to 20 mg Fa/ml are added to a suspension of 50 mg of collagen dissolved in 30 ml of acetic acid solution with 10 mM HEPES. It is stabilised at pH 8 with NaOH and finally adding 10 mg of EDC and allowed to stir for 12 hours. The resulting mixture is stabilised again at pH 3 and allowed to stir for 72 hours at 4°C and then sonicated for 5 minutes and subject to ultracentrifugation on an iodoxinadol bed for 10 hours at 25000 RPM. The supernatant is extracted, dialysed and finally freeze-dried.

## Claims

1. Implant or gel comprising at least a compound with magnetic functionality that comprises in turn a substrate of at least one enzyme involved in the regeneration of the extracellular matrix and a plurality of magnetic particles.

2. Implant or gel according to claim 1, wherein the magnetic particles are bound by covalent bonds to the substrate.

3. Implant or gel according to any of claims 1 or 2, wherein the magnetic particles are superparamagnetic particles.

4. Implant or gel according to any of claims 1 to 3, wherein the substrate is selected from the group consisting of protein, modified protein, polypeptide, modified polypeptide, glycoprotein, glycosaminoglycan and a combination thereof.

5. Implant or gel according to claim 4, where the substrate is selected from the group consisting of collagen, fibrilin, elastin, laminin, osteocalcin, chondroitin sulphate, hyaluronic acid, osteonectin, osteopontin, gelatine and a combination thereof.

6. Implant or gel according to claim 5, wherein the substrate is selected from the group consisting of collagen, chondroitin sulphate and hyaluronic acid.

7. Compound with magnetic functionality comprising a substrate of at least one enzyme involved in the regeneration of the extracellular matrix and a plurality of magnetic particles, to manufacture an implant or gel to monitor an enzymatic activity involved in the regeneration of the extracellular matrix.

8. Compound with magnetic functionality according to claim 7, wherein the magnetic particles are bound by covalent bonds to the substrate.

9. Compound with magnetic functionality according to any of claims 7 or 8, wherein the magnetic particles are superparamagnetic particles.

10. Compound with magnetic functionality according to any of claims 7 to 9, wherein the substrate is selected from the group consisting of protein, modified protein, polypeptide, modified polypeptide, glycoprotein, glycosaminoglycan and a combination thereof.

11. Compound with magnetic functionality according to claim 10 wherein the substrate is selected from the group consisting of collagen, fibrilin, elastin, laminin, osteocalcin, chondroitin sulphate, hyaluronic acid, osteopontin, gelatine and a combination thereof.

12. Compound with magnetic functionality according to claim 11, wherein the substrate is selected from the group consisting of collagen chondroitin sulphate and hyaluronic acid.

13. Use of the compound with magnetic functionality as described in any of claims 7 to 12 to manufacture an implant or gel to monitor an enzymatic activity involved in the regeneration of the extracellular matrix.

14. Use of the compound with magnetic functionality according to claim 13 to monitor the regeneration of connective tissue.

15. Use of the compound with magnetic functionality according to claim 13 to monitor the biological integration of an implant in a human being or another living being.

16. Compound with magnetic functionality comprising a substrate of at least one enzyme and a plurality of magnetic particles, **characterised in that**

i) the magnetic particles do not have any coating or modification of surface,
or
ii) the magnetic particles are functionalised with carboxylic groups.

17. Compound with magnetic functionality according to claim 16, wherein the magnetic particles are superparamagnetic particles.

18. compound with magnetic functionality according to any of claims 16 or 17, wherein the magnetic particles do not have any coating or modification of surface.

19. Compound with magnetic functionality according to any of claims 16 to 18 wherein the substrate is selected from the group consisting of protein, modified protein, polypeptide, modified polypeptide, glycoprotein, glycosaminoglycan and a combination thereof.

20. Compound with magnetic functionality according to claim 19, wherein the substrate is selected from the group consisting of collagen, fibrilin, elastin, laminin, osteocalcin, chondroitin sulphate, hyaluronic acid, osteonectin, osteopontin, gelatine and a combination thereof.

21. Compound with magnetic functionality according to claim 20, wherein the substrate is selected from the group consisting of collagen, chondroitin sulphate and hyaluronic acid.

**22.** Compound with magnetic functionality according to any of claims 16 or 17, wherein the magnetic particles tend to aggregate at physiological pH.

**23.** Compound with magnetic functionality according to any of claims 16 or 17, wherein the magnetic particles have modifications only in part of their surface.

**24.** Method for obtaining a compound with magnetic functionality as described in any of claims 18 to 21, **characterised in that** said method comprises:

a) modifying the substrate of at least one enzyme, and
b) immobilising a plurality of magnetic particles that do not have any coating or modification of surface in the modified compound obtained in stage a).

**25.** Method for obtaining a compound with magnetic functionality according to claim 24, wherein in stage a) the substrate reacts with an aminosilane in an organic medium with a maximum water content of 2%.

**26.** Method for obtaining a compound with magnetic functionality according to claim 25, wherein in stage a) the substrate reacts with an aminosilane that is (3-aminopropyl)-triethoxysilane in an organic medium.

**27.** Method for obtaining a compound with magnetic functionality according to any of claims 25 to 26, wherein the method comprises an additional stage, prior to stage a) of activation of the amino group of aminosilane with glutaric anhydride and N,N-diisopropylethylamine in an organic medium.

**28.** Product comprising at least a compound with magnetic functionality as described in any of claims 16 to 24, wherein said product is selected from the group consisting of an implant, a gel, a pharmaceutical composition and a contrast agent.

**29.** Method according to claim 28, wherein said product is an implant or a gel as described in any of claims 1 to 6.

**30.** Use of the compound with magnetic functionality as described in any of claims 16 to 23 to manufacture a product as described in any of claims 28 to 29 to establish or discriminate the enzymatic activity of one or more enzymes.

**31.** Use of the compound with magnetic functionality according to claim 30 to manufacture an adequate contrast agent to detect a disease or disorder associated with the enzymatic activity of at least one enzyme.

**32.** Use of the compound with magnetic functionality according to claim 31, wherein the disease or disorder is selected from the group consisting of rheumatic diseases, arthritis, lesion, disease related to connective tissue, Dupuytren disease, Peyronie disease, disease related to collagen, steatosis, fibrosis, cirrhosis, metastasis, tissue regeneration, cancer, coronary disease, liver disease, metabolic disease, infection and inflammatory disease.

**33.** Use of the compound with magnetic functionality as described in any of claims 16 to 23 to manufacture an implant or gel to monitor regeneration of the cell matrix.

**34.** Use of the compound with magnetic functionality according to claim 33 to manufacture an implant or gel to monitor cartilage regeneration.

**35.** Use of the compound with magnetic functionality as described in any of claims 13 to 15 or 30 to 34, wherein the enzyme reaction causes at least a partial change of superparamagnetism or paramagnetism.

**36.** Use of the compound with magnetic functionality according to claim 35, wherein the determination of the variation in the magnetic property caused by the enzyme reaction is performed by a technique selected from the group consisting of magnetic resonance imaging, nuclear magnetic resonance, electronic paramagnetic resonance, magnetometry, Mössbauer spectrometry, superconductive quantum interference equipment magnetometry, vibrational magnetometry, X-ray magnetic circular dichroism, ferromagnetic resonance, magneto optic Kerr effect, Brillouin light scattering, electromagnetic inductance, atomic force microscopy, magnetorelaxometry and a combination thereof.

**37.** Use of the compound with magnetic functionality according to claim 36, wherein the determination of the variation in the magnetic property caused by the enzyme reaction I performed by magnetic resonance imaging.

**38.** Use of the compound with magnetic functionality according to claim 37, wherein the variation in the magnetic property caused by the enzyme reaction is determined by a parameter selected from the group consisting of signal intensity, longitudinal relaxation time (T1), transversal relaxation time (T2), diffusion parameter, signal of response to a first series of pulses or saturation signal I and a combination thereof.

**39.** Use of the compound with magnetic functionality according to claim 38, wherein the variation in the magnetic property caused by the enzyme reaction is determined by analysing said parameter before and after the enzyme reaction.

**40.** Use of the compound with magnetic functionality according to any of claims 35 to 39, wherein the variation in a magnetic property is indicative of at least one physical phenomenon selected from the group consisting of:

- aggregation of magnetic particles,
- aggregation of the compound with magnetic functionality or at least one of the products of reaction thereof,
- change in the size of magnetic particles, and
- change in the size of particle of at least one selected from the group consisting of the compound with magnetic functionality subject of the present invention, at least one the products of reaction thereof and a recombination thereof.

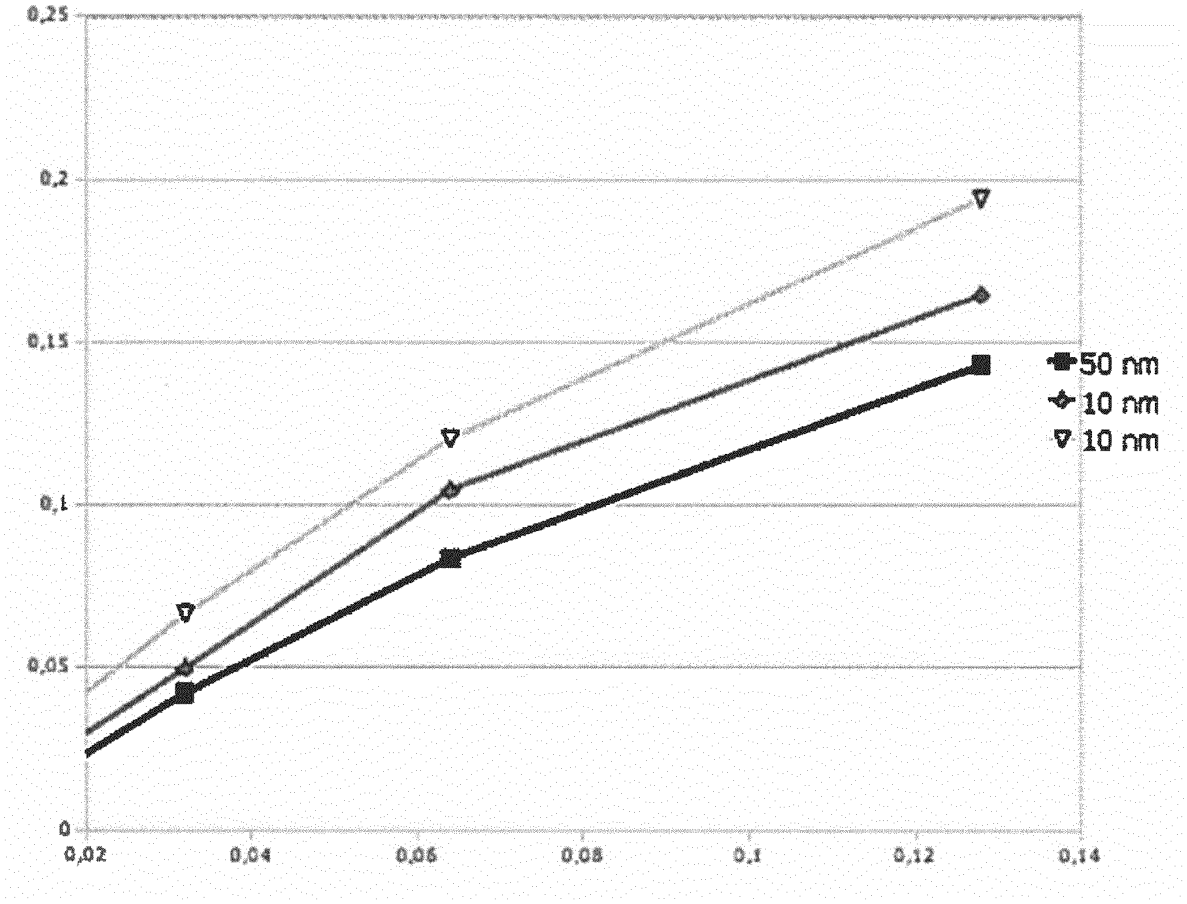


**Fig. 1**

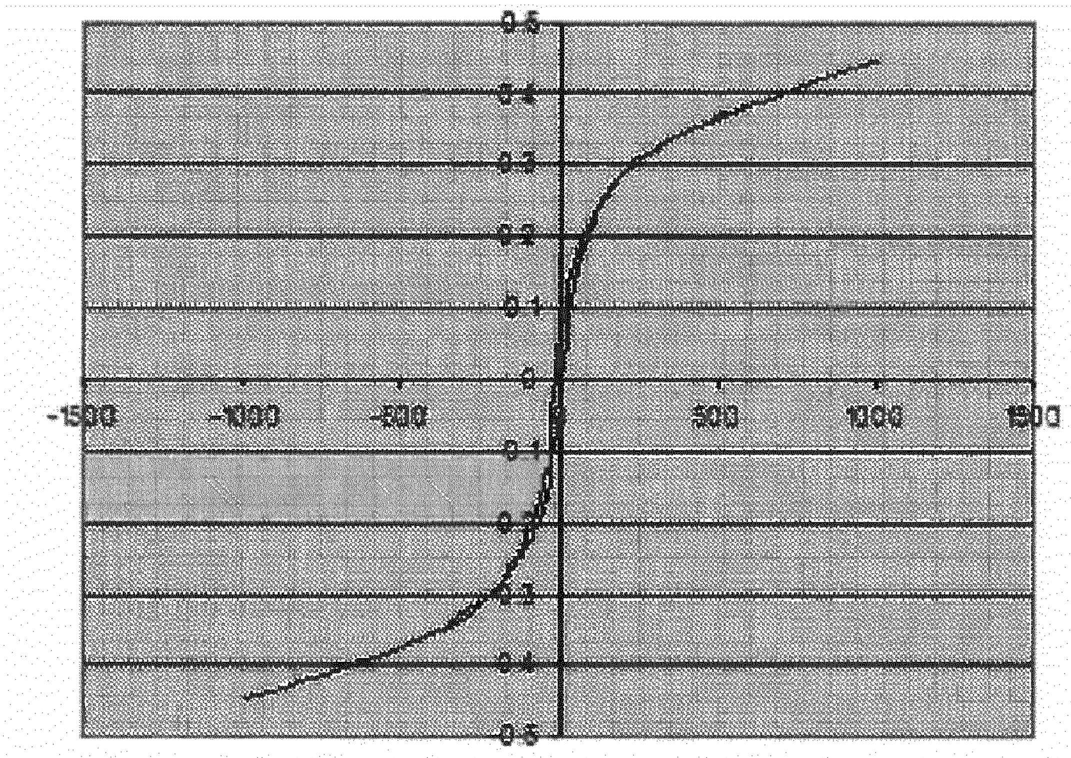


Fig. 2

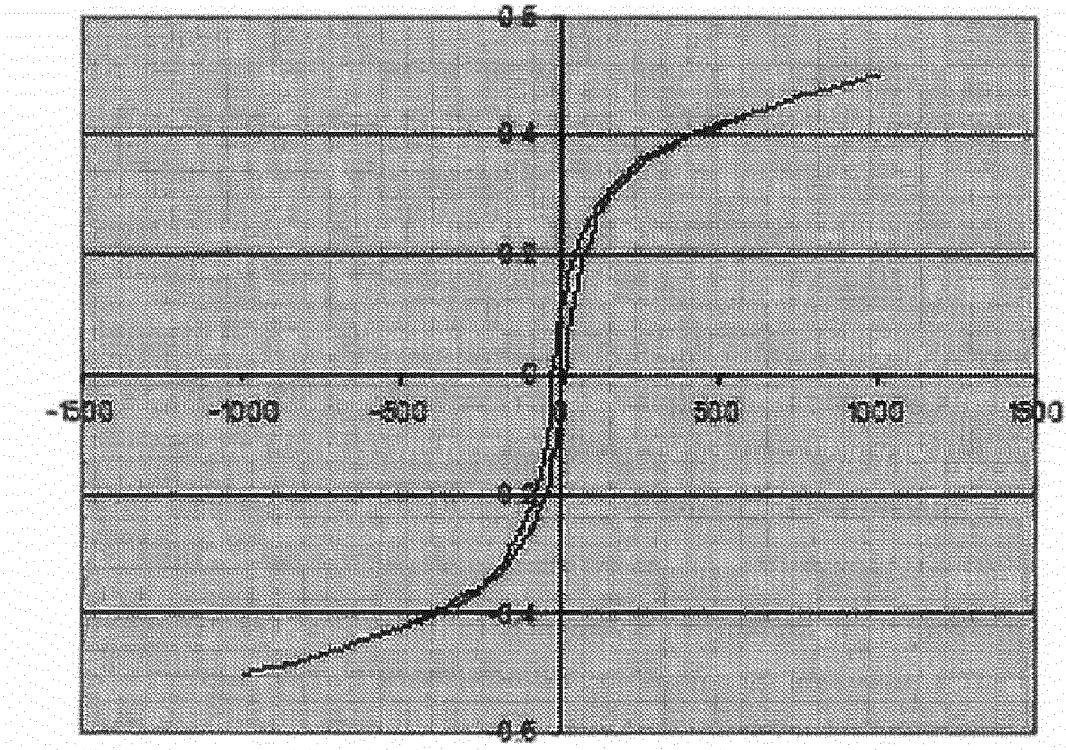


Fig. 3

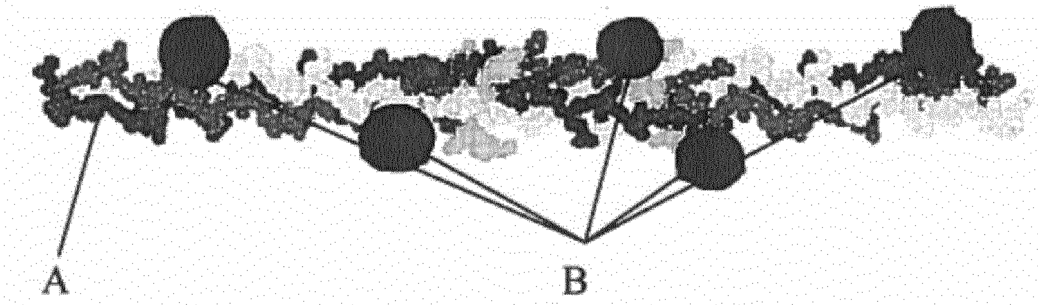


Fig. 4

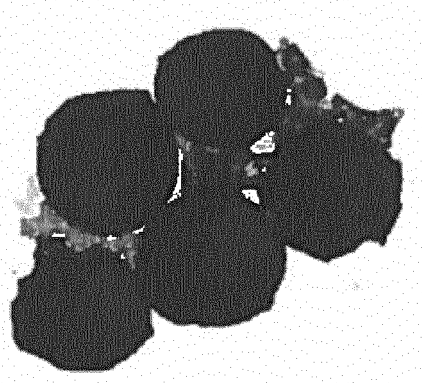

Fig. 5

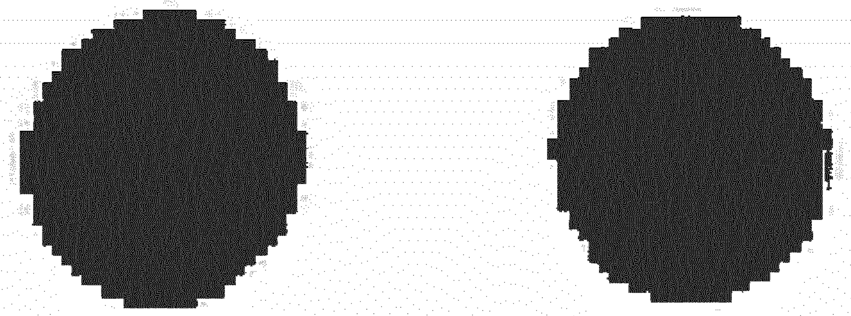

C D

Fig. 6

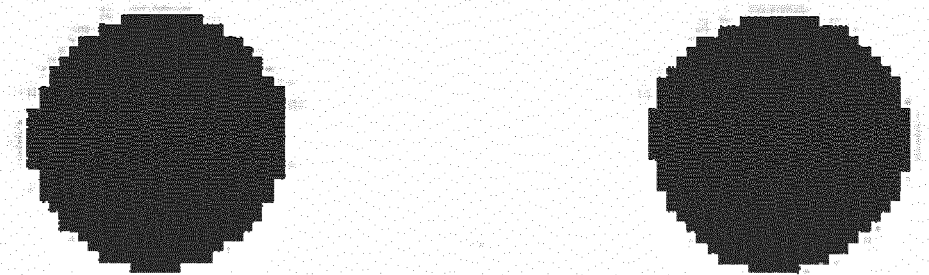

E F

Fig. 7

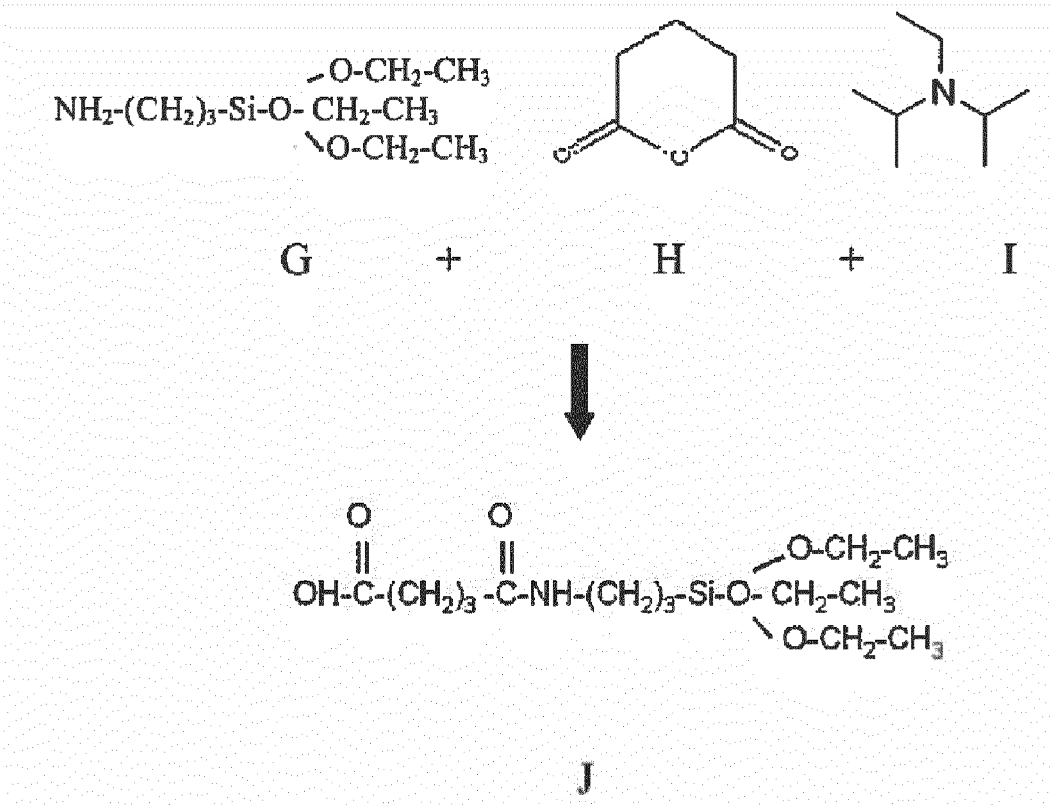

G + H + I

J

Fig. 8

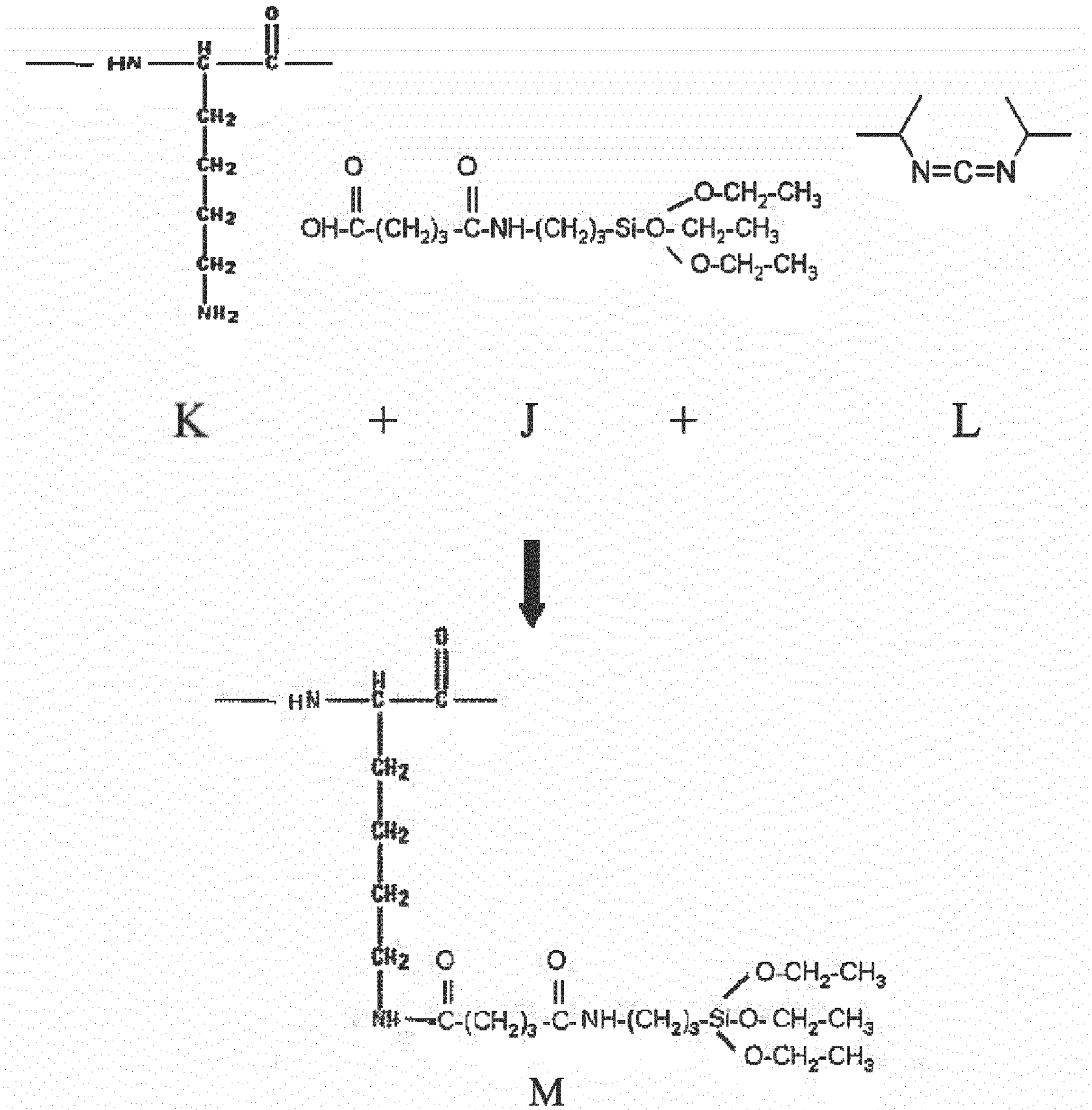

K + J + L

M

**Fig. 9**

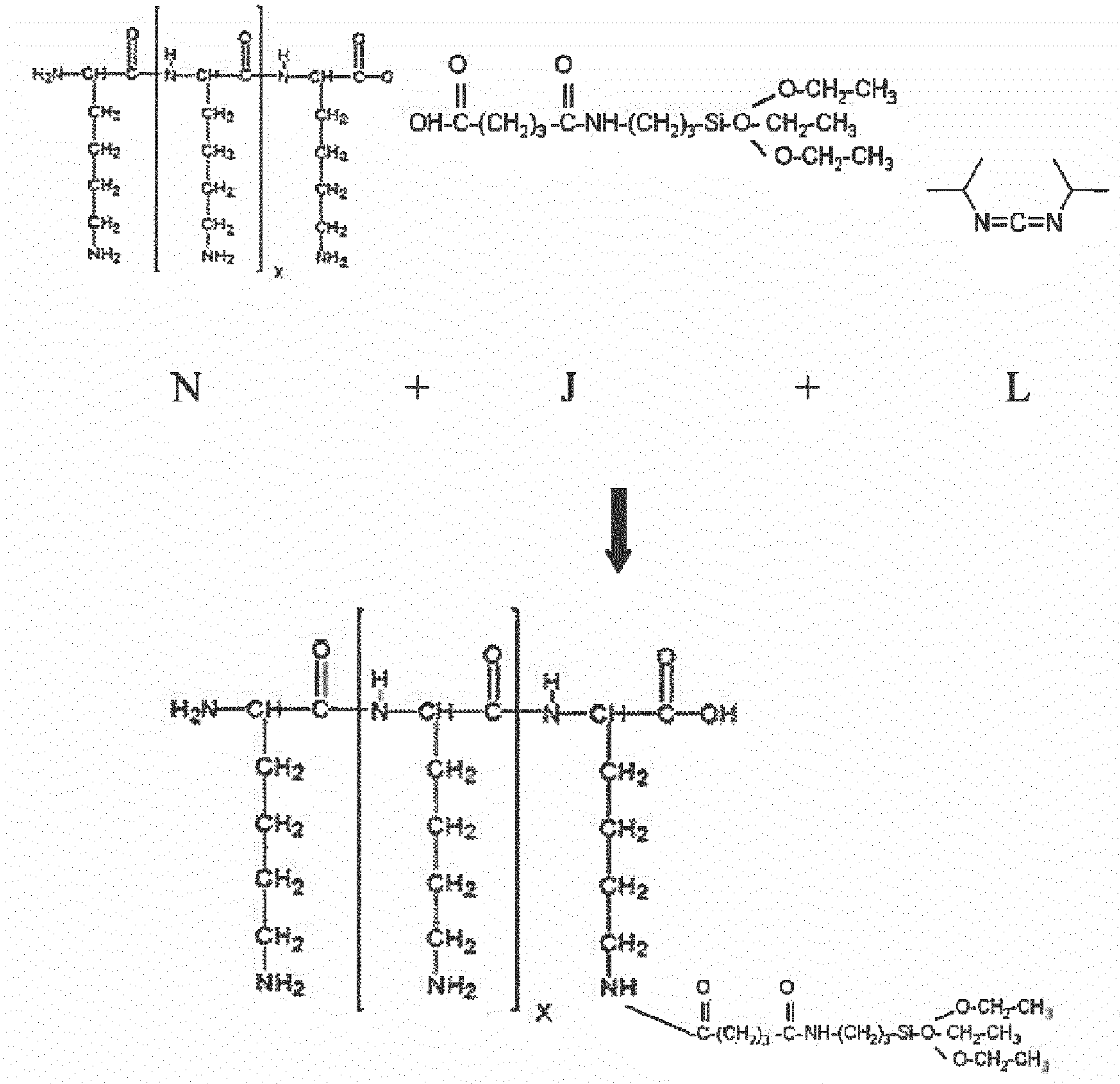

N + J + L

Ñ

**Fig. 10**

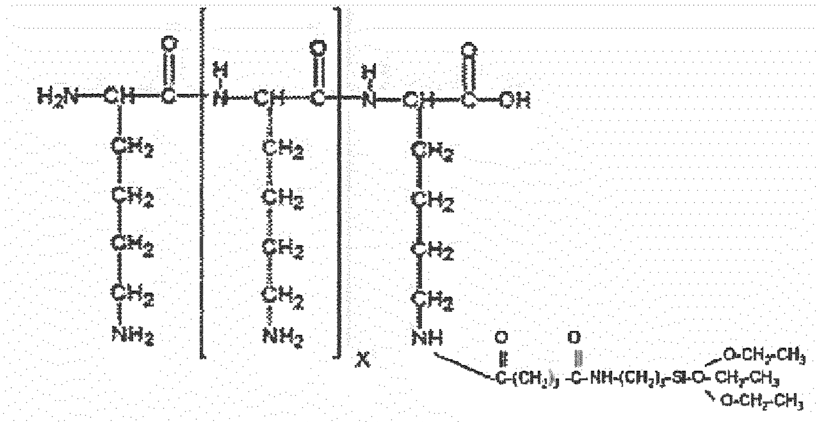

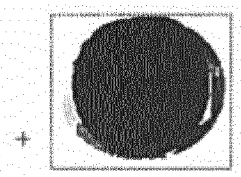

Ñ O

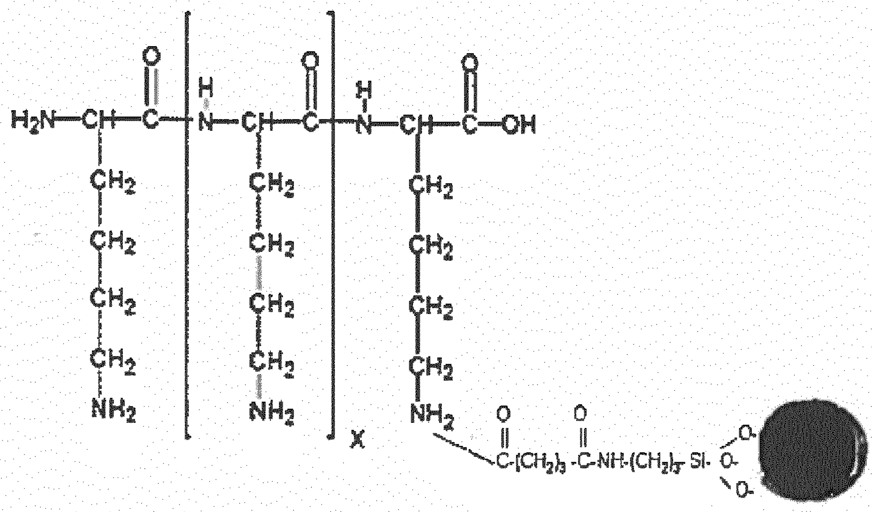

R

Fig. 11

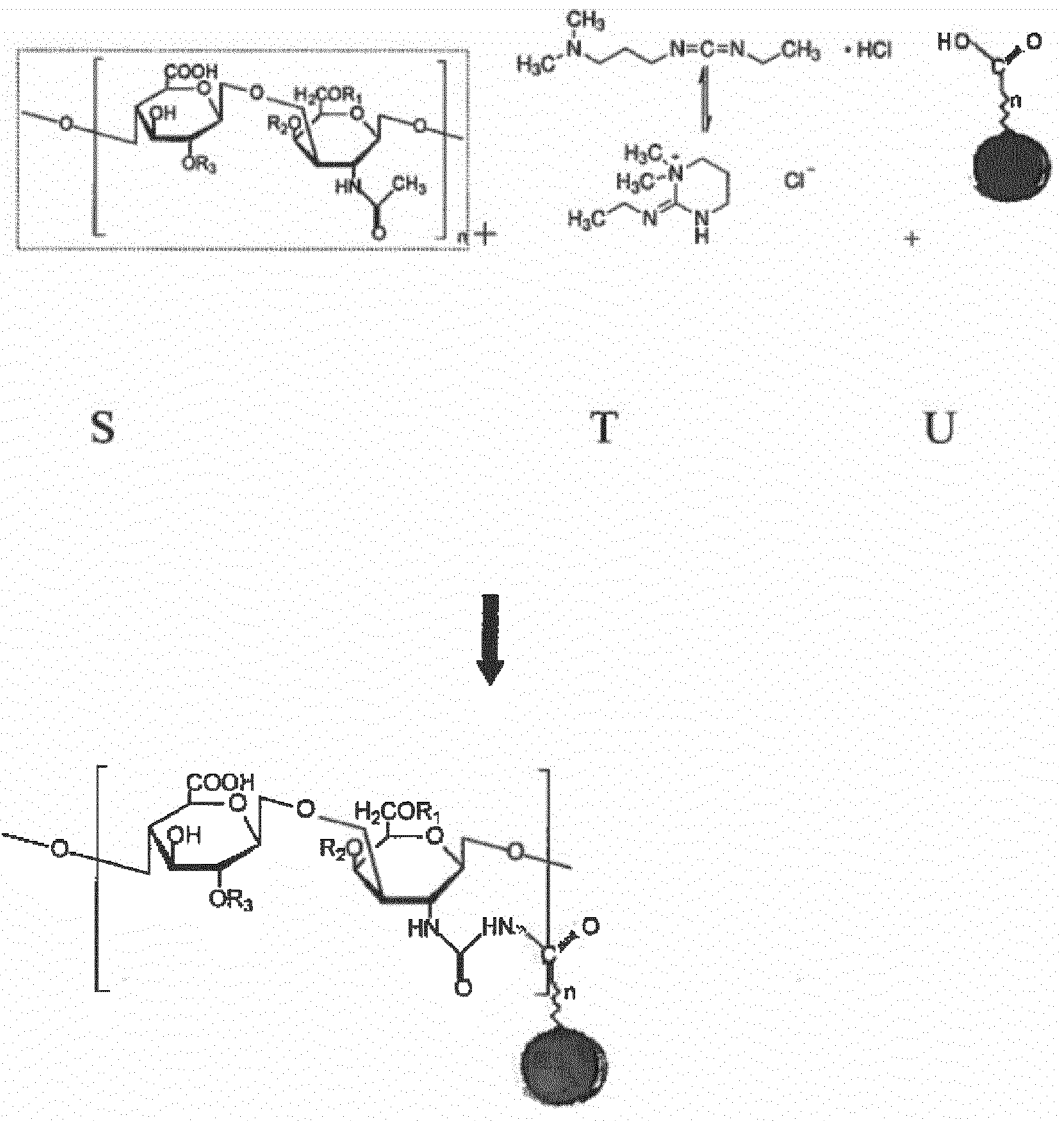

S T U

Fig. 12

# INTERNATIONAL SEARCH REPORT

International application No.
PCT/ES2013/070407

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)
A61K, G01N

Documentation searched other than minimum documentation to the extent that such documents are included in the fields searched

Electronic data base consulted during the international search (name of data base and, where practicable, search terms used)

EPODOC, INVENES, WPI, BIOSIS, EMBASE, NPL, MEDLINE, XPEPSP

C. DOCUMENTS CONSIDERED TO BE RELEVANT

| Category* | Citation of document, with indication, where appropriate, of the relevant passages | Relevant to claim No. |
|---|---|---|
| X | US 2011182815 A1 (DAICH JULIAN) 28/07/2011, Paragraphs [0019], [0021]-[0033], [0036], [0046], [0076], [0080], [0097], [0112]-[0115], abstract and claim 1. | 1-40 |
| A | WO 2011075476 A1 (UNIV ARIZONA ET AL.) 23/06/2011, Paragraphs [0010], [0012]-[0014], [0015], [0017]-[0019]. | 1-40 |
| A | US 2003170173 A1 (KLAVENESS JO ET AL.) 11/09/2003, Paragraphs [0001], [0002], [0035]-[0042], [0088], [0092], [0093] and [0096]-[0098]. | 1-40 |
| A | US 2012107229 A1 (HUANG XUEFEI ET AL.) 03/05/2012, Paragraphs [0001], [0004], [0005], [0013] and [0089]. | 1-40 |
| A | US 2007166810 A1 (BOBROW MARK N ET AL.) 19/07/2007, Paragraphs [0002], [0006]-[0010], [0095] and [0127]. | 1-40 |

☐ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 10/10/2013 | (16.10.2013) |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | S. González Peñalba<br><br>Telephone No. 91 3493025 |

Form PCT/ISA/210 (second sheet) (July 2009)

## INTERNATIONAL SEARCH REPORT

Information on patent family members

International application No.
PCT/ES2013/070407

| Patent document cited in the search report | Publication date | Patent family member(s) | Publication date |
|---|---|---|---|
| US2011182815 A1 | 28.07.2011 | NONE | |
| WO2011075476 A1 | 23.06.2011 | US2013022548 A1 | 24.01.2013 |
| US2003170173 A1 | 11.09.2003 | WO0189584 A2 | 29.11.2001 |
| | | WO0189584 A3 | 02.05.2002 |
| | | JP2003534297 A | 18.11.2003 |
| | | EP1283728 A2 | 19.02.2003 |
| | | AU7468301 A | 03.12.2001 |
| US2012107229 A1 | 03.05.2012 | WO2010120905 A2 | 21.10.2010 |
| | | WO2010120905 A3 | 31.03.2011 |
| US2007166810 A1 | 19.07.2007 | WO2007075910 A2 | 05.07.2007 |
| | | WO2007075910 A3 | 06.12.2007 |
| | | WO2007075891 A2 | 05.07.2007 |
| | | WO2007075891 A3 | 28.02.2008 |
| | | US2007166835 A1 | 19.07.2007 |
| | | EP1969372 A2 | 17.09.2008 |
| | | EP1969372 A4 | 22.04.2009 |
| | | EP1969337 A2 | 17.09.2008 |
| | | EP1969337 A4 | 27.01.2010 |

Form PCT/ISA/210 (patent family annex) (July 2009)

## INTERNATIONAL SEARCH REPORT

International application No.

PCT/ES2013/070407

CLASSIFICATION OF SUBJECT MATTER

***A61K49/14*** (2006.01)
***A61K49/18*** (2006.01)
***G01N33/53*** (2006.01)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20110182815 A1, Daich, J.
- WO 2011075476 A1, Bennet K.


### Non-patent literature cited in the description


- **LACONTE LE ; NITIN N ; ZURKIYA O ; CARUNTU D ; O'CONNOR CJ ; HU X ; BAO G.** *J Magn Reson Imaging.,* December 2007, vol. 26 (6), 1634-41
- **A. ROCH ; F. MONKY ; R.N. MULLER ; P. GILLIS.** *Magnetic Resonance in Colloids an Interface Science,* 383-92
- **MIN-JUNG KIM ; DAE-HWAN JANG ; YONG-HO CHOAE.** *Physica Scripta T,* 2010, vol. 139, 14024
- Macromolecular drug delivery: methods and protocols. **JOHN M. WALKER.** Methods in molecular biology. Springer protocols, vol. 480
- **WOESSNER JF JR.** Matrix metalloproteinases and their inhibitors in connective tissue remodelling. *FASEB J.,* May 1991, vol. 5 (8), 2145-54
- **SOMERS KD ; DAWSON DM.** Fibrin deposition in Peyronie's disease plaque. *J. Urol,* 1997, vol. 157 (1), 311-5